(19) Europäisches Patentamt
European Patent Office
Office européen des brevets

(11) Publication number: **0 550 924 A1**

# (12) EUROPEAN PATENT APPLICATION

(21) Application number: **92203810.4**

(22) Date of filing: **08.12.92**

(51) Int. Cl.5: **C07D 207/26**, C07D 401/04, C07D 401/12, C07D 403/12, C07D 405/04, C07D 405/12, C07D 405/14, C07D 409/04, C07D 409/12, C07D 409/14

(30) Priority: **16.12.91 US 807911**

(43) Date of publication of application: **14.07.93 Bulletin 93/28**

(84) Designated Contracting States: **CH DE FR GB IT LI NL**

(71) Applicant: **MERCK & CO. INC.**
**126, East Lincoln Avenue P.O. Box 2000**
**Rahway New Jersey 07065-0900(US)**

(72) Inventor: **Vacca, Joseph P.**
**766 Eisenhauer Drive**
**Telford, PA 18969(US)**
Inventor: **Ghosh, Arun K.**
**853 Jackson Street**
**Lansdale, PA 19446(US)**
Inventor: **Chen, Lin-Tsen Jenny**
**106 East Rose Valley**
**Wallingsford, PA 19086(US)**
Inventor: **Hungate, Randall W.**
**1925 Rampart Lane**
**Lansdale, PA 19446(US)**

(74) Representative: **Barrett-Major, Julie Diane et al**
**Merck & Co., Inc. European Patent Department Terlings Park Eastwick Road**
**Harlow Essex CM20 2OR (GB)**

## (54) 2-Pyrrolidinone derivatives as HIV protease inhibitors.

(57) Compounds of the form,

A-B-G-J

wherein A is an amine protecting group and the like, B an amino acid or analog thereof, wherein G is

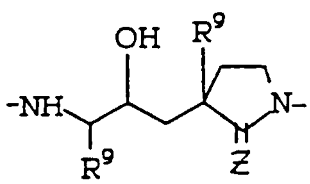

or

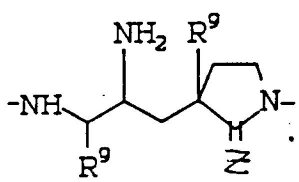

and J a small terminal group are described. These compounds are useful in the inhibition of HIV protease, the prevention or treatment of infection by HIV and the treatment of AIDS, either as compounds, pharmaceutically acceptable salts, pharmaceutical composition ingredients, whether or not in combination with other antivirals, immunomodulators, antibiotics or vaccines. Methods of treating AIDS and methods of preventing or treating infection by HIV are also described.

EP 0 550 924 A1

The present invention is concerned with compounds which inhibit the protease encoded by human immunodeficiency virus (HIV) or pharmaceutically acceptable salts thereof and are of value in the prevention of infection by HIV, the treatment of infection by HIV and the treatment of the resulting acquired immune deficiency syndrome (AIDS). It also relates to pharmaceutical compositions containing the compounds and to a method of use of the present compounds and other agents for the treatment of AIDS & viral infection by HIV.

## BACKGROUND OF THE INVENTION

A retrovirus designated human immunodeficiency virus (HIV) is the etiological agent of the complex disease that includes progressive destruction of the immune system (acquired immune deficiency syndrome; AIDS) and degeneration of the central and peripheral nervous system. This virus was previously known as LAV, HTLV-III, or ARV. A common feature of retrovirus replication is the extensive post-translational processing of precursor polyproteins by a virally encoded protease to generate mature viral proteins required for virus assembly and function. Inhibition of this processing prevents the production of normally infectious virus. For example, Kohl, N.E. et al., Proc. Nat'l Acad. Sci. 85, 4686 (1988) demonstrated that genetic inactivation of the HIV encoded protease resulted in the production of immature, non-infectious virus particles. These results indicate that inhibition of the HIV protease represents a viable method for the treatment of AIDS and the prevention or treatment of infection by HIV.

The nucleotide sequence of HIV shows the presence of a pol gene in one open reading frame [Ratner, L. et al., Nature, 313, 277(1985)]. Amino acid sequence homology provides evidence that the pol sequence encodes reverse transcriptase, an endonuclease and an HIV protease [Toh, H. et al., EMBO J. 4, 1267 (1985); Power, M.D. et al., Science, 231, 1567 (1986); Pearl, L.H. et al., Nature 329, 351 (1987)]. Applicants demonstrate that the compounds of this invention are inhibitors of HIV protease. Compounds in this invention are distinguished by an internal lactam ring in the dipeptide isostere.

## BRIEF DESCRIPTION OF THE INVENTION

Compounds of formula I, as herein defined, are disclosed. These compounds are useful in the inhibition of HIV protease, the prevention of infection by HIV, the treatment of infection by HIV and in the treatment of AIDS, either as compounds, pharmaceutically acceptable salts, pharmaceutical composition ingredients, whether or not in combination with other antivirals, immunomodulators, antibiotics or vaccines. Methods of treating AIDS, methods of preventing infection by HIV, and methods of treating infection by HIV are also disclosed.

## ABBREVIATIONS

| Designation | Amino Acid |
|---|---|
| Ile | D- or L-isoleucine |
| Val | D- or L-valine |
| | **Activating Agent** |
| HBT (HOBT or HOBt) | 1-hydroxybenzotriazole hydrate |
| DEPC | diethylphosphonyl cyanide |
| HOOBT | 3,4-dihydro-3-hydroxy-4-oxo-1,2,3-benzotriazine |

| | |
|---|---|
| | <u>Condensing Agent</u> |
| EDC | 1-ethyl-3-(3-dimethylamino-propyl)carbodiimide |
| DCC | dicyclohexylcarbodiimide |
| | <u>Deprotonating Agents</u> |
| n-BuLi | n-butyllithium |
| LDA | lithium diisopropylamide |
| LHMDS | lithium hexamethyldisilylazane |
| SHMDS | sodium hexamethyldisilylazane |
| | <u>Other Reagents</u> |
| $BF_3{\bullet}OEt_2$ | boron trifluoride etherate |
| TEA | triethylamine |

## DETAILED DESCRIPTION OF THE INVENTION AND PREFERRED EMBODIMENTS

This invention is concerned with the compounds of formula I, combinations thereof, or pharmaceutically acceptable salts thereof, in the inhibition of HIV protease, the prevention or treatment of infection by HIV and in the treatment of the resulting acquired immune deficiency syndrome (AIDS). Compounds of formula I are defined as follows:

A-B-G-J  I,

wherein A is:

1) trityl,
2) hydrogen;
3)

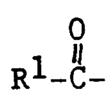

wherein $R^1$ is

a) hydrogen,
b) $C_{1-4}$ alkyl, substituted with one or more halogens adjacent to the carbonyl carbon where halogen is F, Cl, Br, and I,
c) aryl unsubstituted or substituted with one or more of
    i) $C_{1-4}$ alkyl,
    ii) $C_{1-3}$ alkoxy,
    iii) halo,
    iv) nitro,
    v) acetoxy,
    vi) dimethylaminocarbonyl,
    vii) phenyl,
    viii) $C_{1-3}$ alkoxycarbonyl, or
    ix) hydroxy,
d) fluorenyl,

e) a 5-7 membered heterocycle such as pyridyl, furyl or benzisoxazolyl, substituted or unsubstituted with one or more of
i) $C_{1-4}$ alkyl,
ii) $C_{1-3}$ alkoxy,
iii) halo,
iv) nitro,
v) acetoxy,
vi) dimethylaminocarbonyl,
vii) phenyl,
viii) $C_{1-3}$ alkoxycarbonyl, or
ix) hydroxy,
f) indole, quinolyl, naphthyl, benzofuryl, or 4-oxo-benzopyranyl,
g) a 5-7 membered cycloalkyl group unsubstituted or substituted with one or more of
i) $C_{1-4}$ alkyl,
ii) $C_{1-3}$ alkoxy,
iii) halo,
iv) nitro,
v) acetoxy,
vi) dimethylaminocarbonyl,
vii) phenyl,
viii) $C_{1-3}$ alkoxycarbonyl, or
ix) hydroxy,
4) phthaloyl wherein the aromatic ring is unsubstituted or substituted with one or more of
a) $C_{1-4}$ alkyl,
b) halo,
c) hydroxy,
d) nitro,
e) $C_{1-3}$ alkoxy,
f) $C_{1-3}$ alkoxycarbonyl,
g) cyano,
h)

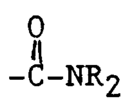

wherein R is H or $C_{1-4}$ alkyl;
5)

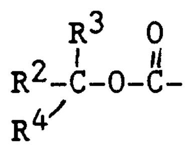

wherein $R^2$,$R^3$, and $R^4$ are independently
a) H,
b) $C_{1-6}$ alkyl unsubstituted or substituted with one or more of
i) halo,
ii) alkyl $SO_2$-,
iii) aryl $SO_2$-,
c) Aryl unsubstituted or substituted with one or more of
i) $C_{1-4}$ alkyl,
ii) $C_{1-3}$ alkoxy,
iii) halo,
iv) nitro,
v) acetoxy,

vi) dimethylaminocarbonyl,
vii) phenyl,
viii) $C_{1-3}$ alkoxycarbonyl

d) fluorenyl,

e) $R^2$, $R^3$, and $R^4$ may be independently joined to form a monocyclic, bicyclic, or tricyclic ring system which is $C_{3-10}$ cycloalkyl and may be substituted with $C_{1-4}$ alkyl,

f) a 5-7 membered heterocycle such as pyridyl, furyl, or benzisoxazolyl;

6)

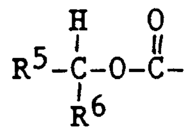

wherein $R^5$ and $R^6$ are

a) $C_{1-4}$ alkyl,

b) aryl,

c) $R^5$ and $R^6$ are joined to form a 5-7 membered heterocycle;

7)

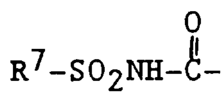

wherein $R^7$ is aryl unsubstituted or substituted with one or more of

a) $C_{1-4}$ alkyl,

b) halo,

c) nitro,

d) $C_{1-3}$ alkoxy;

8)

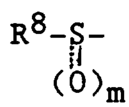

wherein m is 0-2 and $R^8$ is

a) $R^7$ as defined above,

b) trityl;

9)

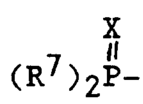

wherein X is O, S or NH, and $R^7$ is defined above;

B is, independently, absent or

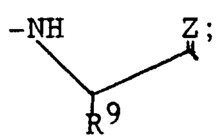

G is

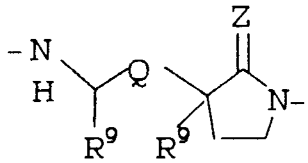

wherein Z is O, S, or NH and
Q is

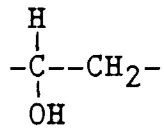

or

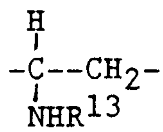 ;

$R^9$ is independently
1) Hydrogen;
2)

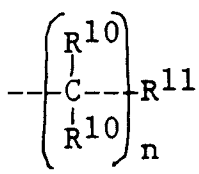 ;

3) $-OR^{11}$,
4) $-N(R^{11})_2$,
5) $C_{1-4}$ alkylene-$R^{11}$ or
6) $-S(R^{11})$;

wherein n is 0-5 and $R^{10}$ is independently
a) hydrogen,
b) hydroxy, or
c) $C_{1-4}$-alkyl;

$R^{11}$ is
a) hydrogen,
b) $C_6$-$C_{10}$ aryl, unsubstituted or substituted with one or more of
i) halo,
ii) hydroxy,
iii) $-NH_2$, $-NO_2$, -NHR, or $-NR_2$,
wherein R is
H, or $C_{1-4}$ alkyl,
iv) $C_{1-4}$ alkyl,
v) $C_{1-3}$ alkoxy,
vi) -COOR,
vii)

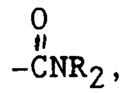

viii) $-CH_2NR_2$,

ix)

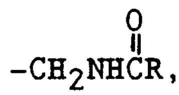

x) CN,

xi) $CF_3$,

xii)

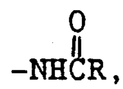

xiii) aryl $C_{1-3}$ alkoxy,

xiv) aryl,

xv) $-NRSO_2R$,

xvi) $-OP(O)(OR_x)_2$ wherein $R_x$ is H or aryl,

xvii)

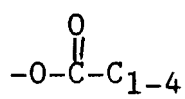

alkyl substituted with one or more of amine or quaternary amine, or

xviii) $-R^{12}$, as defined below:

c) 5 or 6 membered heterocycle including up to 3 heteroatoms selected from N, O, and S, such as imidazolyl, thiazolyl, furyl, oxazolyl, piperidyl, thiadiazolyl, piperazinyl, pyridyl, or pyrazinyl, any of which heterocycle may be unsubstituted or substituted with one or more of

i) halo,

ii) hydroxy,

iii) $-NH_2$, -NHR, $-NR_2$,

iv) $C_{1-4}$ alkyl,

v) $C_{1-3}$ alkoxy,

vi) -COOR,

vii)

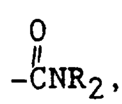

viii) $-CH_2NR_2$,

ix)

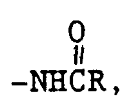

x) -CN,

xi) $CF_3$,

xii) $-NHSO_2R$,
xiii) $-OP(O)(OR_x)2$ wherein $R_x$ is H or aryl,
xiv)

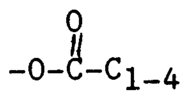

alkyl substituted with one or more of amine or quaternary amine, or
xv) $-R^{12}$;
d) $C_{1-6}$ alkyl or $C_{1-6}$ alkenyl,
unsubstituted or substituted with one or more of
i) hydroxy,
ii) $C_{1-4}$ alkyl,
iii) $-NH_2$, -NHR, $-NR_2$,
iv)

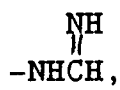

v)

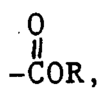

vi) -SR, or arylthio,
xi) $-SO_2NHR$,
vii) $C_{1-4}$ alkyl sulfonyl amino or aryl sulfonyl amino,
viii) -CONHR,
ix)

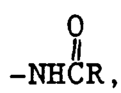

x) -OR,
xi) aryl $C_{1-3}$ alkoxy,
xii) aryl, or
xiii) aryl substituted with $R^{12}$;
e) $C_{3-7}$ cycloalkyl unsubstituted or substituted with one or more of
i) hydroxy,
ii) $C_{1-4}$alkyl,
iii) $-NH_2$, -NHR, $-NHR_2$,
iv)

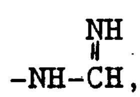

v)

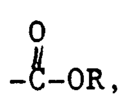

vi) -SR,
vii) $-SO_2NH_2$,
viii) alkyl sulfonylamino or aryl sulfonylamino,
ix) -CONHR,
x)

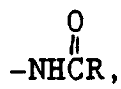

or
xi) $-R^{12}$;

f) a 5- to 7-membered carbocyclic or 7- to 10-membered bicyclic carbocyclic ring which is either saturated or unsaturated, such as cyclopentane, cyclohexane, indan, norbornane, or naphthalene, the carbocyclic ring being unsubstituted or substituted with one or more of
i) halo
ii) -OR, wherein R is H or $C_{1-4}$ alkyl,
iii)

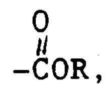

iv)

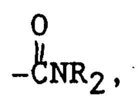

v) $-CH_2NR_2$,
vi) $-SO_2NR_2$ or $-S(O)_y$ R wherein y is 0,1 or 2,
vii) $-NR_2$,
viii)

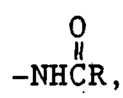

ix) $C_{1-4}$ alkyl,
x) phenyl,
xi) $-CF_3$,
xii)

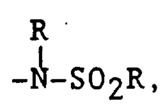

or
xiii) $-R^{12}$;

$R^{12}$ is
a) $-X-(CH_2)_m--XR^{13}$ where X is independently -O-,-S-, or NR; m is 2-5, and $R^{13}$ is independently hydrogen or
i) $C_{1-6}$ alkyl,
ii) $C_{1-6}$ alkyl substituted with one or more of
(a) $C_{1-3}$ alkoxy,
(b) -OH,
(c) $-NR_2$ where R is hydrogen or $C_{1-4}$ alkyl;
iii) aromatic heterocycle unsubstituted or substituted with one or more of

(a) $C_{1-4}$ alkyl, or
(b) $-NR_2$;

b) $-X-(CH_2)_m-NR^{13}R^{13}$ wherein $R^{13}$ is the same or different and joined together to form a 5-7 member heterocycle containing up to two additional heteroatoms selected from

(a) -NR,
(b) -O-,
(c) -S-,
(d)

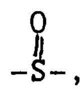

(e) $-SO_2-$;

c) $-(CH_2)_q--NR^{13}R^{13}$ wherein q is 1-5, and $R^{13}$ is defined above;

J is

1) $R^{14}$ wherein:
$R^{14}$ is

a) H;
b) $C_{1-6}$ alkyl, unsubstituted or substituted with one or more of

i) $-NR_2$,
ii) -OR,
iii) $-NHSO_2C_{1-4}$ alkyl,
iv) $-NHSO_2$ aryl, or $-NHSO_2$(dialkylaminoaryl),
v) $-CH_2OR$,
vi) $-C_{1-4}$ alkyl,
vii)

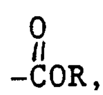

viii)

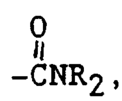

ix)

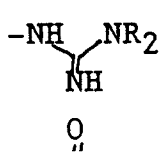 ; 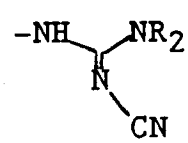

x)

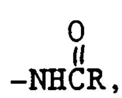

xi)

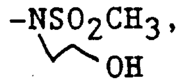

xii)

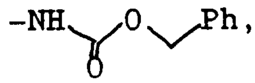

xiii) $-NR_3^{\oplus}A^{\ominus}$ wherein $A^{\ominus}$ is a counterion,
xiv) $-NR^{15}R^{16}$ wherein $R^{15}$ and $R^{16}$ are the same or different and are $C_{1-5}$ alkyl joined together directly to form a 5-7 membered heterocycle,
xv) aryl,
xvi) -CHO,
xvii) $-OP(O)(OR_x)_2$ wherein $R_x$ is H or aryl, or
xvii)

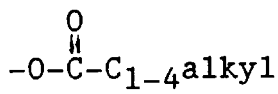

substituted with one or more of amine or quaternary amine;
c) $-(CH_2CH_2O)_nCH_3$ or $-(CH_2CH_2O)_nH$;
2)

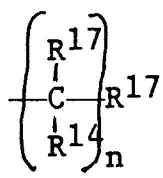

wherein:
$R^{14}$ and n are defined above, and
$R^{17}$ is
a) hydrogen;
b) aryl unsubstituted or substituted with one or more of
i) halo,
ii) -OR, wherein R is H or $C_{1-4}$ alkyl,
iii)

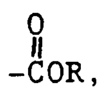

iv)

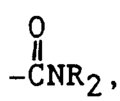

v) $-CH_2NR_2$,
vi) $-SO_2NR_2$,
vii) $-NR^2_2$,
viii)

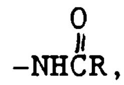

xi) $C_{1-4}$ alkyl,
x) phenyl
xi) $-CF_3$,
xii)

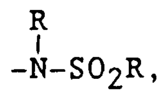

xiii) $-C_{1-4}$ alkyl $-NR_2$,
xiv) $-OP(O)(OR_x)_2$ wherein $R_x$ is H or aryl, or
xv)

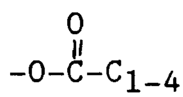

alkyl substituted with one or more of amine or quaternary amine;

c) Heterocycle, unsubstituted or substituted with one or more of
i) halo,
ii) -OR, wherein R is H, $C_{1-4}$alkyl, or $C_{1-4}$alkenyl,
iii)

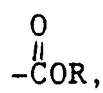

iv)

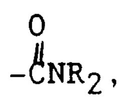

v) $-CH_2NR_2$,
vi) $-SO_2NR_2$,
vii) $-NR_2$,
viii)

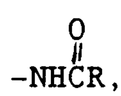

xi) $C_{1-4}$ alkyl,
x) phenyl
xi) $-CF_3$,
xii)

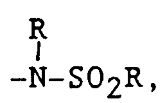

xiii) phenyl $C_{1-4}$ alkyl,
xiv) $-OP(O)(OR_x)_2$ wherein $R_x$ is H or aryl,
xv)

$$-O-\overset{O}{\overset{\|}{C}}-C_{1-4}$$

alkyl substituted with one or more of amine or quaternary amine;

d) A 5 to 7 membered carbocyclic or 7-10 membered bicyclic carbocyclic ring which is either saturated or unsaturated, such as cyclopentane, cyclohexane, indan, norbornane, or naphthalane, the carbocyclic ring being unsubstituted or substituted with one or more of

i) halo,
ii) -OR, wherein R is H or $C_{1-4}$ alkyl,
iii)

$$-\overset{O}{\overset{\|}{C}}OR,$$

iv)

$$-\underset{O}{\underset{\|}{C}}NR_2,$$

v) $-CH_2NR_2$,
vi) $-SO_2NR_2'$,
vii) $-NR_2$,
viii)

$$-NH\overset{O}{\overset{\|}{C}}R,$$

xi) $C_{1-4}$ alkyl,
x) phenyl
xi) $-CF_3$,
xii)

$$-\overset{R}{\overset{/}{N}}-SO_2R,$$

xiii) $-OP(O)(OR_x)_2$ wherein $R_x$ is H or aryl, or
xiv)

$$-O-\overset{O}{\overset{\|}{C}}-C_{1-4}$$

alkyl substituted with one or more of amine, quaternary amine, or $-OP(O)(OR_x)2$; or
xv)

$$O\overset{O}{\overset{\|}{C}}O-((CH_2)_mO)_n-R,$$

or pharmaceutically acceptable salts thereof.

In the compounds of the present invention, the A, G, B and J components and the like may have asymmetric centers and occur as racemates, racemic mixtures and as individual diastereomers, with all isomeric forms being included in the present invention.

When any variable (e.g., aryl, heterocycle, R, $R^1$, $R^2$, $R^3$, $R^4$, $R^7$, $R^{10}$, $R^{11}$, $R^{12}$, $R^{15}$, $R^{16}$, $A^{\ominus}$, n, Z, etc.) occurs more than one time in any constituent or in formula I, its definition on each occurrence is independent of its definition at every other occurrence. Also, combinations of substituents and/or variables are permissible only if such combinations result in stable compounds.

As used herein except where noted, "alkyl" is intended to include both branched- and straight-chain saturated aliphatic hydrocarbon groups having the specified number of carbon atoms (Me is methyl, Et is ethyl, Pr is propyl, Bu is butyl); "alkoxy" represents an alkyl group of indicated number of carbon atoms attached through an oxygen bridge; and "cycloalkyl" is intended to include saturated ring groups, such as cyclopropyl, cyclobutyl, cyclopentyl, cyclohexyl (Cyh) and cycloheptyl. "Alkenyl" or "alkylene" is intended to include hydrocarbon claims of either a straight or branched configuration and one or more unsaturated carbon-carbon bonds which may occur in any stable point along the chain, such as ethenyl, propenyl, butenyl, pentenyl, and the like. "Halo", as used herein, means fluoro, chloro, bromo and iodo; and "counterion" is used to represent a small, single negatively-charged species, such as chloride, bromide, hydroxide, acetate, trifluroacetate, perchlorate, nitrate, benzoate, maleate, tartrate, hemitartrate, benzene sulfonate, and the like.

As used herein, with exceptions as noted, "aryl" is intended to mean phenyl (Ph). "Carbocyclic" is intended to mean any stable 5- to 7-membered carbon ring or 7- to 10-membered bicyclic carbon ring, any of which may be saturated or partially unsaturated.

The term heterocycle, as used herein except where noted, represents a stable 5- to 7-membered mono- or bicyclic or stable 7- to 10-membered bicyclic heterocyclic ring which is either saturated or unsaturated, and which consists of carbon atoms and from one to three heteroatoms selected from the group consisting of N, O and S, and wherein the nitrogen and sulfur heteroatoms may optionally be oxidized, and the nitrogen heteroatom may optionally be quaternized, and including any bicyclic group in which any of the above-defined heterocyclic rings is fused to a benzene ring. The heterocyclic ring may be attached at any heteroatom or carbon atom which results in the creation of a stable structure. Examples of such heterocyclic elements include piperidinyl, piperazinyl, 2-oxopiperazinyl, 2-oxopiperidinyl, 2-oxopyrrolodinyl, 2-oxoazepinyl, azepinyl, pyrrolyl, 4-piperidonyl, pyrrolidinyl, pyrazolyl, pyrazolidinyl, imidazolyl, imidazolinyl, imidazolidinyl, pyridyl, pyrazinyl, pyrimidinyl, pyridazinyl, oxazolyl, oxazolidinyl, isoxazolyl, isoxazolidinyl, morpholinyl, thiazolyl, thiazolidinyl, isothiazolyl, quinuclidinyl, isothiazolidinyl, indolyl, quinolinyl, isoquinolinyl, benzimidazolyl, thiadiazoyl, benzopyranyl, benzothiazolyl, benzoxazolyl, furyl, tetrahydrofuryl, tetrahydropyranyl, thienyl, benzothienyl, thiamorpholinyl, thiamorpholinyl sulfoxide, thiamorpholinyl sulfone, and oxadiazolyl.

One embodiment of the compounds of the present invention encompasses those compounds of Formula I in which B is independently present once and Z is O. In this embodiment, it is preferred that Q is

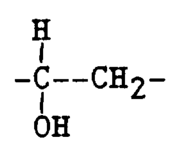 .

A second embodiment of the compounds of the present invention encompasses those compounds of Formula I in which B is absent. In this embodiment, it is preferred that Q is

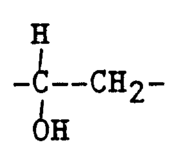 .

A third embodiment of the compounds of the present invention encompasses those compounds of Formula I in which G is

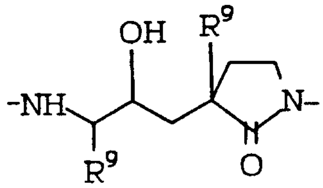 or 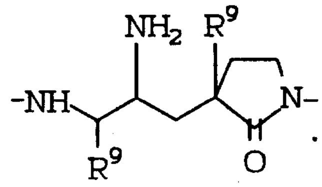 .

A fourth embodiment of the compounds of the present invention encompasses those compounds of Formula I in which G is

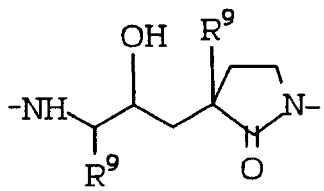 or 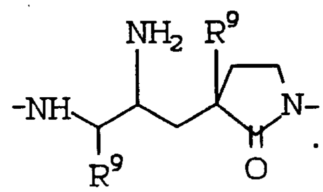 .

B is absent or present once, and
J is

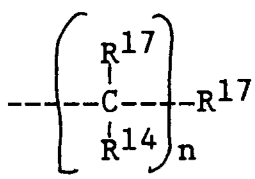 .

A fifth embodiment of the compounds of the present invention encompasses those compounds of Formula I in which
A is

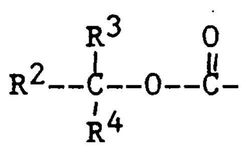 ;

G is

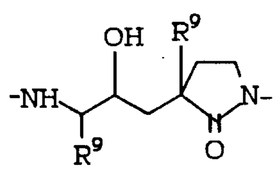

or

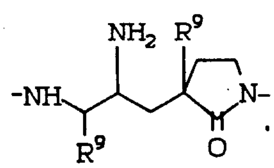 .

B is absent or present once, and
J is

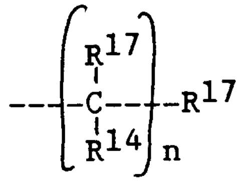 .

A sixth embodiment of the compounds of the present invention encompasses compounds of Formula I wherein
A is

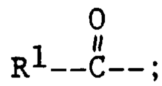

G is

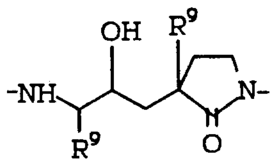 ;

B is absent or present once, and
J is

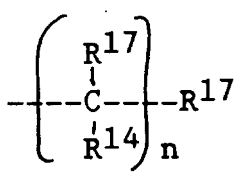 .

Preferred compounds of the invention include the following:
N'-[4(RS)-(3,4-dihydro-1H-2-benzothiopyranylsulfoxide)]-3(S)-[3(S)-((1,1-dimethylethoxycarbonyl)-amino)-2-(S)-hydroxy-4-phenylbutyl]-3(S)-(phenylmethyl)pyrrolidin-2-one,
N'-[4(R)-(3,4-dihydro-1H-2-benzothiopyranylsulfone)]-3(S)-[3(S)-((1,1-dimethylethoxycarbonyl)amino)-2(S)-hydroxy-4-phenylbutyl]-3(S)-(phenylmethyl)-pyrrolidin-2-one,
N'-[4(S)-(3,4-dihydro-1H-2-benzothiopyranylsulfone)]-3(S)-[3(S)-(3(S)-tetrahydrofuranoxycarbonylamino)-2-(S)-hydroxy-4-phenylbutyl]-3(S)-(phenylmethyl)-pyrrolidin-2-one,
N'-[4(RS)-(3,4-dihydro-1H-2-benzothiopyranyl)]-3(S)-[3(S)-((1,1-dimethylethoxycarbonyl)amino)-2(S)-hydroxy-4-cyclohexylbutyl]-3-(S)-(4-hydroxyphenyl-methyl)-pyrrolidin-2-one,
N'-[4(RS)-(3,4-dihydro-1H-2-benzothiopyranyl)]-3(S)-[3(S)-(1,1-dimethylethoxycarbonylamino)-2(S)-hydroxy-4-(cyclohexyl)butyl]-3-(S)-(phenylmethyl)-pyrrolidin-2-one,
N'-[4(S)-(3,4-dihydro-1H-2-benzothiopyranylsulfide)]-3(S)-[3(S)-(3(S)-tetrahydrofuranoxycarbonylamino)-2(S)-hydroxy-4-(cyclohexyl)butyl]-3(S)-(phenylmethyl)-pyrrolidin-2-one,
N'-[4(R)-(3,4-dihydro-1H-2-benzothiopyranylsulfide)]-[3(S)-(3(S)-tetrahydrofuranoxycarbonylamino)-2(S)-hydroxy-4-(phenyl)-butyl]-3(S)-(phenylmethyl)-pyrrolidinone,
N'-[4(RS)-(3,4-dihydro-1H-2-benzothiopyranyl)]-3(S)-[3(S)-(1,1,-dimethylethoxycarbonylamino)-2(S)-hydroxy-4-(cyclohexyl)-butyl]-3(S)-((4-(2-(4-morpholino)-ethoxy)phenyl)methyl)-pyrrolidin-2-one,
N'-[4(R)-(cis(3-hydroxy-1-indanyl))]-3(S)-[3(S)-(3(S)-tetrahydrofuranoxycarbonylamino)-2(S)-hydroxy-4-phenylbutyl]-3(S)-(phenylmethyl)-pyrrolidin-2-one,

N'-[4(S),2(R)-(3,4-dihydro-1H-2-benzooxothiopyranyl)]-3(S)-[3(S)-(3(S)-tetrahydrofuranoxycarbonylamino)-2-(S)-hydroxy-4-(cyclohexyl)butyl]-3(S)-(phenylmethyl)-pyrrolidin-2-one,
N'-[1-hydroxy-3-methyl-2-cyclopentyl]-3(S)-[3(S)-(1,1-dimethylethoxycarbonylamino)-2(S)-hydroxy-4-phenylbutyl]-3(S)-(phenylmethyl)-pyrrolidin-2-one,
N'-[(4S)-(3,4-dihydro-1H-2-benzothiopyranyl)]-3(S)-[3(S)-(3(S)-tetrahydrofuranyloxylcarbonylamino)-2(S)-hydroxy-4-(cyclohexyl)butyl]-3(S)-((4-(2-(4-morpholino)ethoxy)phenyl)methyl)-pyrrolidin-2-one,
N'-[(4S),(2S)-(3,4-dihydro-1H-2-benzooxothiopyranyl)]-3(S)-[3(S)-(3(S)-tetrahydrofuranyloxycarbonylamino)-2-(S)-hydroxy-4-(cyclohexyl)-butyl]-3(S)-(4-hydroxy-phenylmethyl)-pyrrolidin-2-one,
N'-[(4S),(2RS)-(3,4-dihydro-1H-2-benzooxothiopyranyl)] -3(S)-[3(S)-3(S)-tetrahydrofuranyloxycarbonylamino)-2(S)-hydroxy-4-(cyclohexyl)-butyl]-3(S)-((4-(2-(4-morpholino)ethoxy)phenyl)methyl)-pyrrolidin-2-one,
N'-[1-hydroxy-3-methyl-2-cyclopentyl]-3(S)-[3(S)-(3(S)-tetrahydrofuranyloxycarbonylamino)-2(S)-hydroxy-4-phenylbutyl]-3(S)-(phenylmethyl)-pyrrolidin-2-one,
N'-[2(S)-isopropylethanol]-3(S)-[3(S)-(3(S)-tetra-hydrofuranoxycarbonylamino)-2(S)-hydroxy-4-(phenyl)-butyl]-3(S)-(phenylmethyl)-pyrrolidin-2-one,
N'-[(5S, 1'S)-(2-(methyl)ethyl-dihydrofuran-2-(3H)-one)yl]-3(S)-[3(S)-[N-(3(S)-tetrahydrofuranoxy-carbonyl)-amino]-2(S)-hydroxy-4-(phenyl)butyl]-3(S)-phenylmethyl-pyrrolidin-2-one,
N'-[2(S)-isopropylethanol]-3(S)-[3(S)-[N-(4(R)-hydroxy-3(S)-tetrahydrofuranoxycarbonyl)amino]-2(S)-hydroxy-4-phenylbutyl]-3(S)-phenylmethyl-pyrrolidin-2-one,
N'-[1(R)-hydroxy-3(R)-methyl-2-cyclopentyl]-3(S)-[3(S)-[N-(3(S)-tetrahydrofuranoxycarbonyl)amino]-2(S)-hydroxy-4-phenylbutyl]-3(S)-phenylmethyl-pyrrolidin-2-one,
N'-[1(R)-hydroxy-3(R)-methyl-2-cyclopentyl]-3(S)-[(3(S)-[N-(3(S)-tetrahydrofuranoxycarbonyl)amino]-2(S)-hydroxy-4-(cyclohexyl)butyl]-3(S)-((4-(2-(dimethylamino)ethoxy)phenyl)methyl)-pyrrolidin-2-one,
N'-[1(R)-hydroxy-3(R)-methyl-2-cyclopentyl]-3(S)-[(3(S)-[N-(3(S)-tetrahydrofuranoxycarbonyl)amino]-2(S) -hydroxy-4-(cyclohexyl)butyl]-3(S)-((4-(2-(4-morpholino)ethoxy)phenyl)methyl)-pyrrolidin-2-one,
N'-[1(R)-hydroxy-3(R)-methyl-2-cyclopentyl]-3(S)-[3-(S)-[N-(3(S)-tetrahydrofuranoxycarbonyl)amino]-2(S)-hydroxy-4-(cyclohexyl)butyl]-3(S)-((4-(2-(3,6,9,12-tetraoxatridecyloxy)phenyl)methyl)-pyrrolidin-2-one,
N'-[1(R)-hydroxy-3(R)-methyl-2-cyclopentyl]-3(S)-[3-(S)-[(N-(benzyloxycarbonyl)-L-Valinyl)amino]-2(S)-hydroxy-4-phenylbutyl]-3(S)-phenylmethyl-pyrrolidin-2-one,
N'-[1(R)-hydroxy-3(R)-methyl-2-cyclopentyl]-3(S)-[3-(S)-[(N-(2-quinolylcarbonyl)-L-Valinyl)amino]-2(S)-hydroxy-4-phenylbutyl]-3(S)-phenylmethyl-pyrrolidin-2-one,
N'-[1(R)-hydroxy-3(R)-methyl-2-cyclopentyl]-3(S)-[3-(S)-[(N-(benzyloxycarbonyl)-L-Valinyl)amino]-2(S)-hydroxy-4-(cyclohexyl)butyl]-3(S)-((4-(2-(4-morpholino)ethoxy)phenyl)methyl)-pyrrolidin-2-one,
N'-[1(R)-hydroxy-3(R)-methyl-2-cyclopentyl]-3(S)-[3-(S)-[(N-(2-quinolylcarbonyl)-L-valinyl)amino]-2(S)-hydroxy-4-(cyclohexyl)butyl]-3(S)-((4-(2-(4-morpholino)ethoxy)phenyl)methyl)-pyrrolidin-2-one,
N'-[1(R)-hydroxy-3(R)-methyl-2-cyclopentyl]-3(S)-[3-(S)-[(N-(4-pyridylcarbonyl)-L-valinyl)amino]-2(S)-hydroxy-4-phenylbutyl]-3(S)-phenylmethyl-pyrrolidin2-one,
N'-[1(R)-hydroxy-3(R)-methyl-2-cyclopentyl]-3(S)-[3-(S)-[(N-(3-pyridylcarbonyl)-L-valinyl)amino]-2(S)-hydroxy-4-phenylbutyl]-3(S)-phenylmethyl-pyrrolidin2-one,
N'-[1(R)-hydroxy-3(R)-methyl-2-cyclopentyl]-3(S)-[3-(S)-[(N-(4-oxo-4H-1-benzopyran-2-carbonyl)-L-valinyl)-amino]-2(S)-hydroxy-4-phenylbutyl]-3(S)-phenylmethylpyrrolidin-2-one,
N'-[1(R)-hydroxy-3(R)-methyl-2-cyclopentyl]-3(S)-[3-(S)-[(N-(4-morpholinocarbonyl)-L-valinyl)amino]-2(S)-hydroxy-4-phenylbutyl]-3(S)-phenylmethyl-pyrrolidin2-one,
N'-[1(R)-hydroxy-3(R)-methyl-2-cyclopentyl]-3(S)-[3-(S)-[(N-(3-pyridylcarbonyl)-L-valinyl)amino]-2(S)-hydroxy-4-(cyclohexyl)butyl]-3(S)-((4-(2-(4-morpholino)ethoxy)phenyl)methyl)-pyrrolidin-2-one,
N'-[1(R)-hydroxy-3(R)-methyl-2-cyclopentyl]-3(S)-[3-(S)-[(N-(4-oxo-4H-1-benzopyran-2-carbonyl)-L-valinyl)-amino]-2(S)-hydroxy-4-(cyclohexyl)butyl]-3(S)-((4-(2-(4-morpholino)ethoxy)phenyl)methyl-pyrrolidin-2-one,
N'-[(5S, 1'S)-(2-(methyl)ethyl-dihydrofuran-2-(3H)-one)yl]-3(S)-[3(S)-[(N-(4-oxo-4H-1-benzopyran-2-carbonyl)-L-valinyl)amino]-2(S)-hydroxy-4-(cyclohexyl)-butyl]-3(S)-((4-(2-(4-morpholino)ethoxy)phenyl)methyl-pyrrolidin-2-one, or
N'-[(5S, 1'S)-(2-(methyl)ethyl-dihydrofuran-2-(3H)-one)yl]-3(S)-[3(S)-[(N-(3-pyridylcarbonyl)-L-valinyl)amino]-2(S)-hydroxy-4-(cyclohexyl)butyl]-3(S)-phenylmethylpyrrolidin-2-one,
or pharmaceutically acceptable salt or ester thereof.

The most preferred compounds include the following:

A:

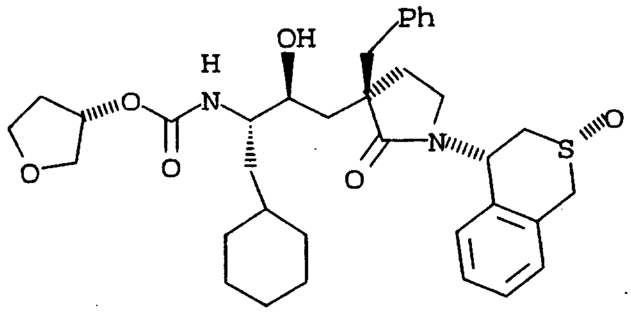

N'-[4(S),2(R)-(3,4-dihydro-1H-2-benzooxothiopyranyl)]-3(S)-[3(S)-(3(S)-tetrahydrofuranoxycarbonyl-amino)-2-(S)-Hydroxy-4-(cyclohexyl)butyl] -3(S)-(phenylmethyl)-pyrrolidin-2-one;

B:

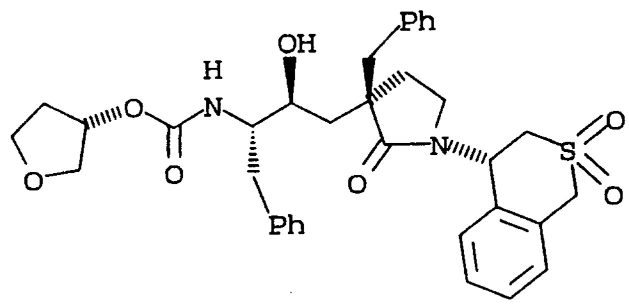

N'-[4(S)-(3,4-dihydro-1H-2-benzothiopyranylsulfone)]-3(S)-[3(S)-(3(S)-tetrahydrofuranoxycarbonyl-amino)-2-(S)-Hydroxy-4-(phenylbutyl)]-3(S)-(phenylmethyl)-pyrrolidin-2-one;

C:

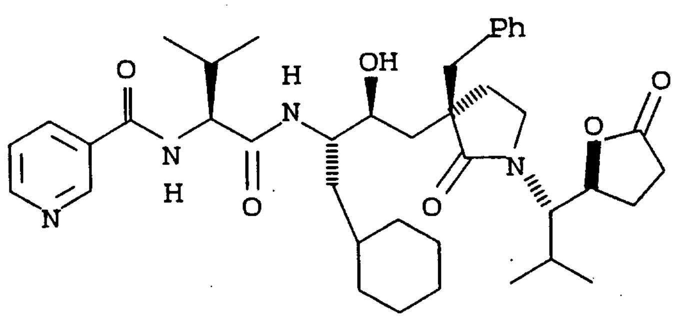

N'-[(5S,1'S)-(2-(methyl)ethyl-dihydrofuran-2-(3H)-one)yl]-3(S)-[3(S)-[(N-(3-pyridylcarbonyl)-L-valinyl) amino]-2(S)-hydroxy-4-(cyclohexyl)butyl]-3(S)-phenylmethyl-pyrrolidin-2-one;

D:

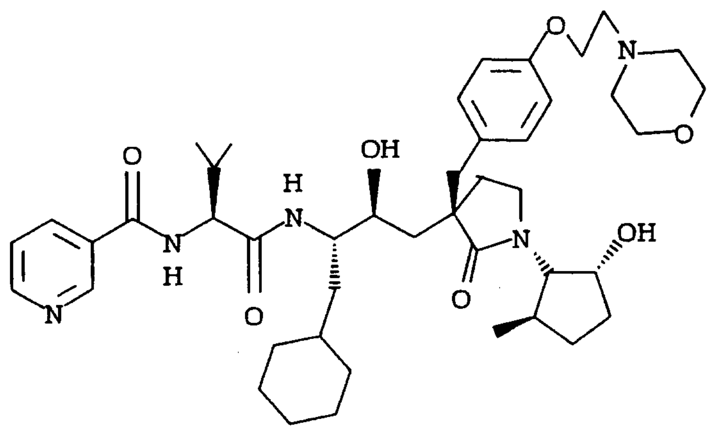

N'-[1(R)-hydroxy-3(R)-methyl-2-cyclopentyl]-3(S)-[3(S) -[(N-(3-pyridylcarbonyl)-L-valinyl)amino]-2(S)-hydroxy -4-(cyclohexyl)butyl]-3(S)-((4-(2-(4-morpholino) ethoxy)phenyl)methyl)-pyrrolidin-2-one;

E:

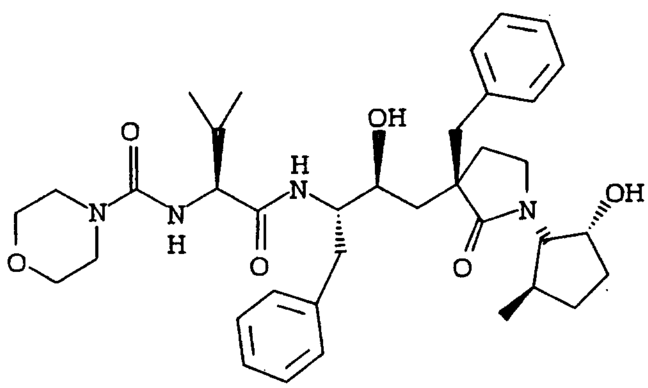

N'-[1(R)-hydroxy-3(R)-methyl-2-cyclopentyl]-3(S)-[3-(S)-[(N-(4-morpholinocarbonyl)-L-valinyl)amino]-2(S)-hydroxy-4-phenylbutyl]-3(S)-phenylmethyl-pyrrolidin-2-one;

F:

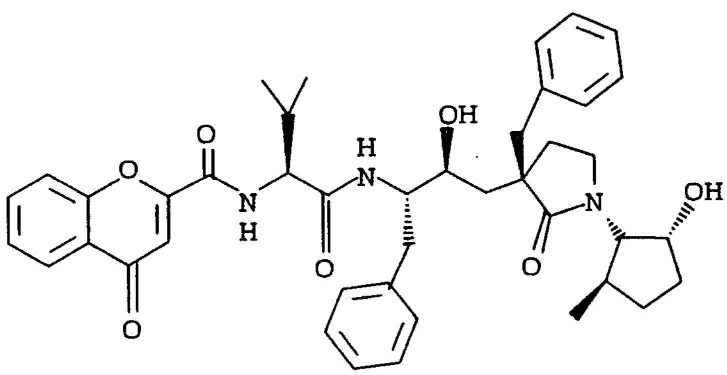

N'-[1(R)-hydroxy-3(R)-methyl-2-cyclopentyl]-3(S)-[3(S) -[(N-(4-oxo-4H-1-benzopyran-2-carbonyl)-L-valinyl) amino]-2(S)-hydroxy-4-phenylbutyl]-3(S)-phenylmethyl -pyrrolidin-2-one;

G:

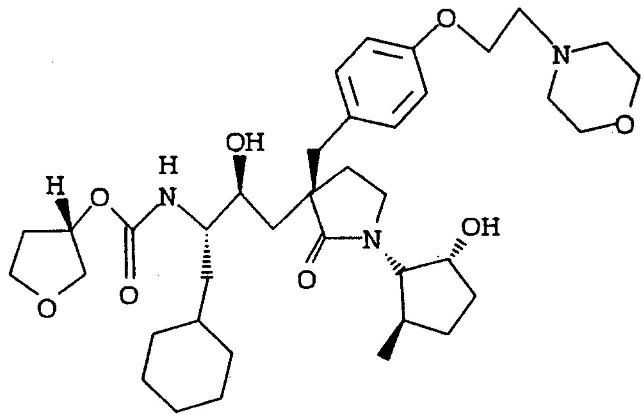

N'-[1(R)-hydroxy-3(R)-methyl-2-cyclopentyl]-3(S)-[3-(S)-[N-(3(S)-tetrahydrofuranoxycarbonyl)amino]-2-(S)-hydroxy-4-(cyclohexyl)butyl]-3(S)-((4-(2-(4-morpholino)ethoxy)phenyl)methyl)-pyrrolidin-2-one;
H:

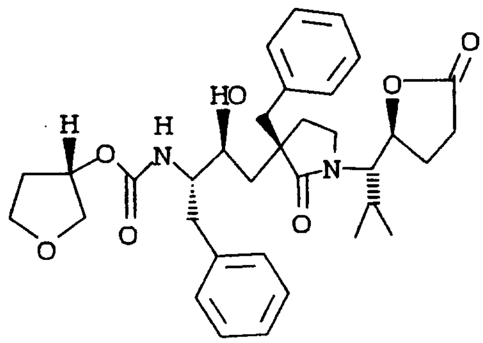

N'-[(5S,1'S)-(2-(methyl)ethyl-dihydrofuran-2-(3H)-one)yl]-3(S)-[3(S)-[N-(3(S)-tetrahydrofuranoxy-carbonyl)-amino]-2(S)-hydroxy-4-(phenyl)butyl]-3(S)-phenylmethyl-pyrrolidin-2-one,
or pharmaceutically acceptable salt thereof.

The pharmaceutically-acceptable salts of the compounds of Formula I (in the form of water- or oil-soluble or dispersible products) include the conventional non-toxic salts or the quaternary ammonium salts of these peptides, which are formed, e.g., from inorganic or organic acids or bases. Examples of such acid addition salts include acetate, adipate, alginate, aspartate, benzoate, benzene-sulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenyl-propionate, picrate, pivalate, propionate, succinate, tartrate, thiocyanate, tosylate, and undecanoate. Base salts include ammonium salts, alkali metal salts such as sodium and potassium salts, alkaline earth metal salts such as calcium and magnesium salts, salts with organic bases such as dicyclohexylamine salts, N-methyl-D-glucamine, and salts with amino acids such as arginine, lysine, and so forth. Also, the basic nitrogen-containing groups may be quaternized with such agents as lower alkyl halides, such as methyl, ethyl, propyl, and butyl chloride, bromides and iodides; dialkyl sulfates like dimethyl, diethyl, dibutyl; and diamyl sulfates, long chain halides such as decyl, lauryl, myristyl and stearyl chlorides, bromides and iodides, aralkyl halides like benzyl and phenethyl bromides and others.

HIV protease inhibitors of Formula I may be prepared in accordance with well-known procedures for preparing peptide analogs from their constituent amino acids or analogs thereof. In general, once the G substituent is made, the rest of the synthesis follows the principle of amide bond formation by the coupling methods of either solution-phase or solid-phase peptide synthesis. The addition and removal of one or more protecting groups is also typical practice.

## Scheme I

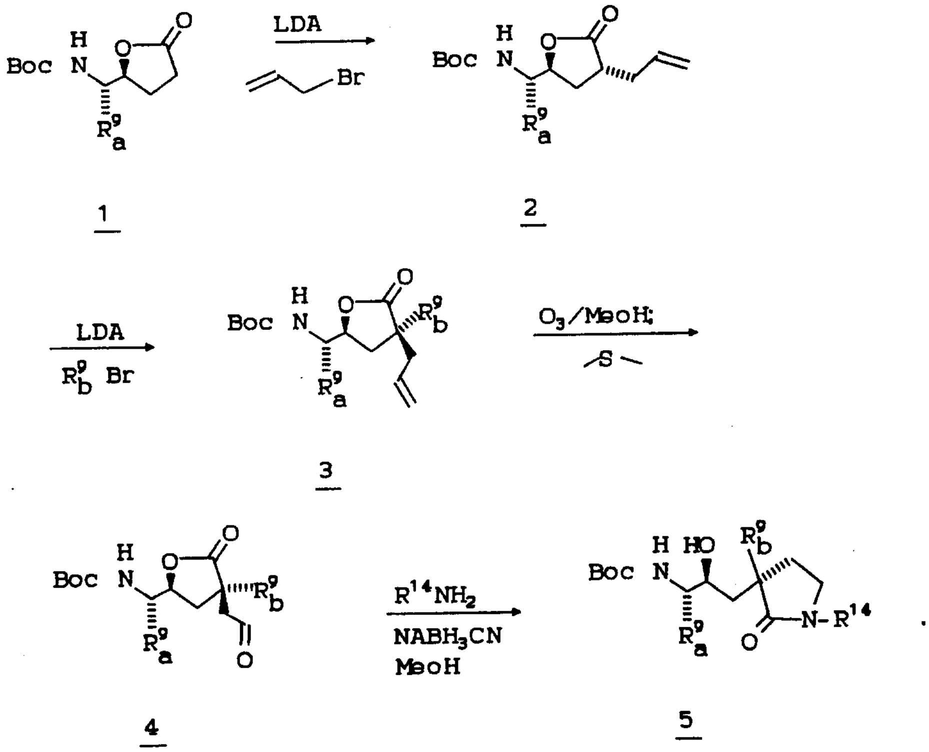


1 2 3 4 5

Scheme I is illustrated by, but not limited to, the compounds of Table I.

Scheme II

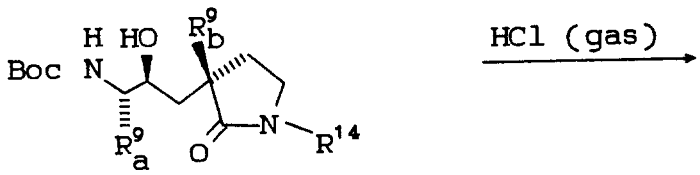


5

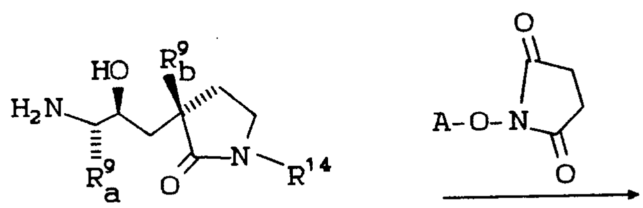

6

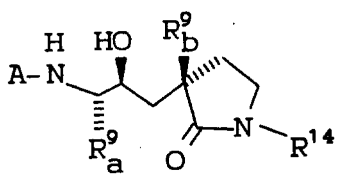

7

Scheme II is illustrated by, but not limited to, the compounds of Table II.

Scheme III

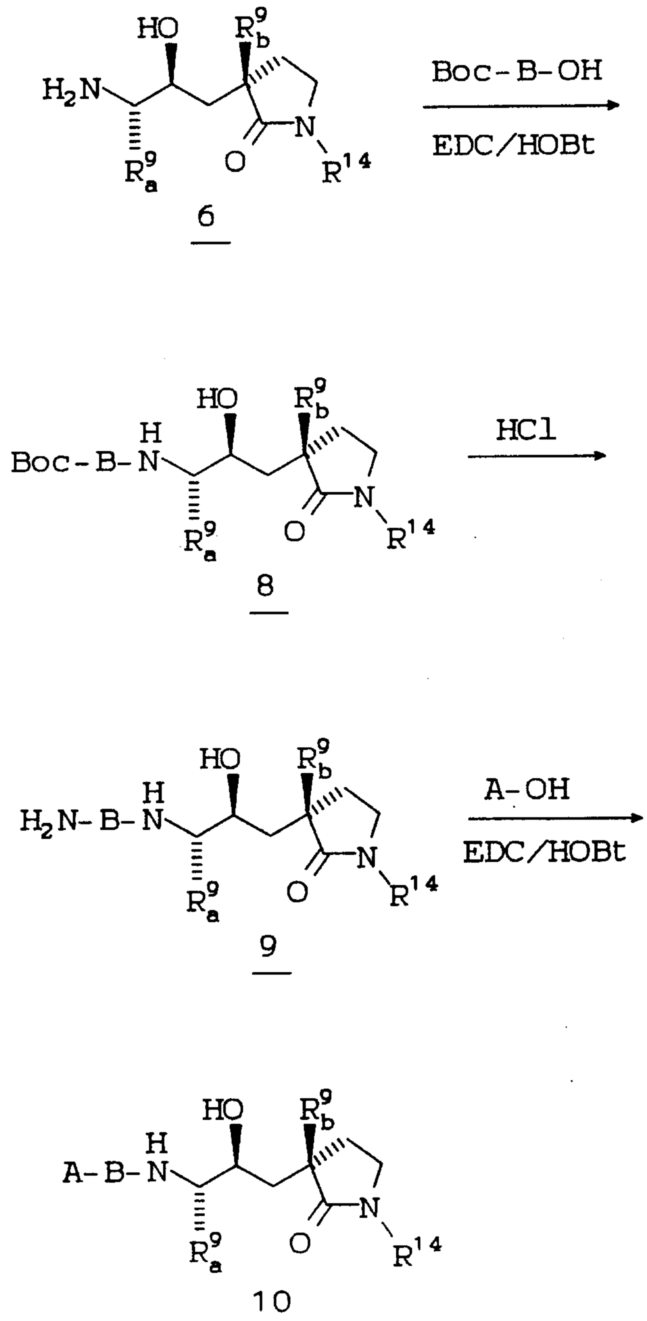


6

8

9

10

Scheme III is illustrated by, but not limited to, the compounds of Table III.

Scheme IV

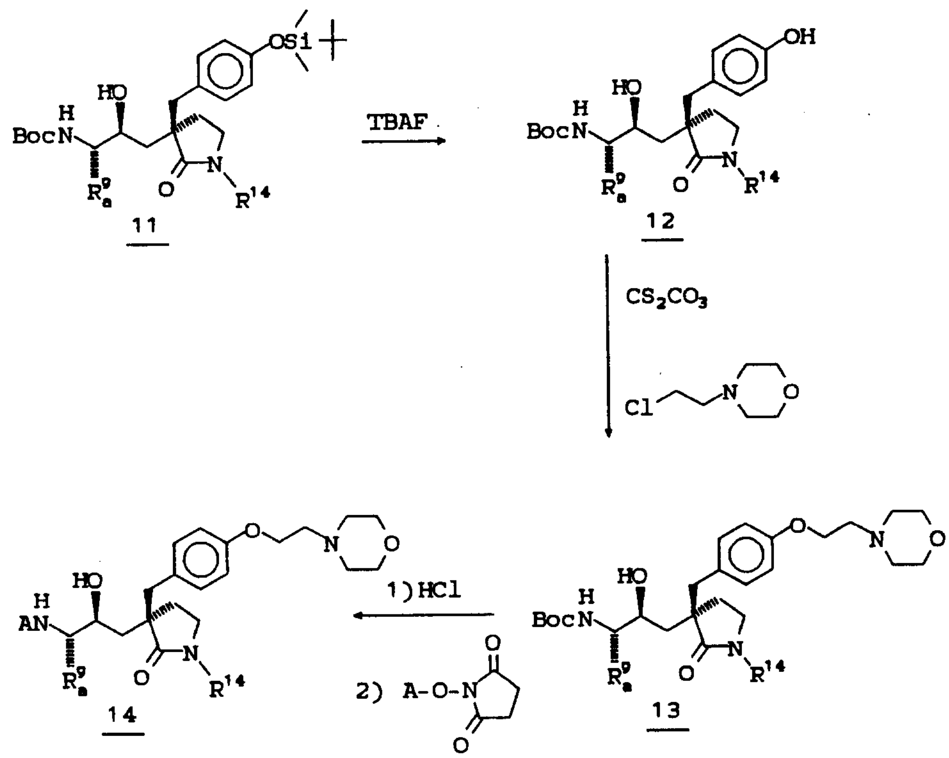


11 12 13 14

Amide couplings used to form the compounds of this invention are typically performed by the carbodiimide method with reagents such as dicyclohexylcarbodiimide, or N-ethyl, N'-(3-dimethylaminopropyl) carbodiimide. Other methods of forming the amide or peptide bond include, but are not limited to synthetic routes via an acid chloride, azide, mixed anhydride or activated ester. Typically, solution phase amide couplings are performed, but solid-phase synthesis by classical Merrifield techniques may be employed instead.

The selection of protecting groups is, in part, dictated by particular coupling conditions, in part by the amino acid and peptide components involved in the reaction. Such amino-protecting groups ordinarily employed include those which are well known in the art, for example, urethane protecting substituents such as benzyloxycarbonyl (carbobenzoxy), p-methoxycarbobenzoxy, p-nitrocarbobenzoxy, t-butyloxycarbonyl, and the like. It is preferred to utilize t-butyloxycarbonyl (BOC) for protecting the $\alpha$-amino group in the amino acids undergoing reaction at the carboxyl end of said amino acid, in part because the BOC protecting group is readily removed by relatively mild acids such as trifluoroacteic acid (TFA), or hydrogen chloride in ethyl acetate.

The OH group of Thr, Tyr or Ser and analogs thereof may be protected by the Bzl (benzyl) group and the epsilon-amino group of Lys may be protected by the IPOC group or the 2-chlorobenzyloxycarbonyl (2-C1-CBZ) group. Treatment with HF or catalytic hydrogenation are typically employed for removal of IPOC or 2-C1-CBZ.

The following Tables specifically illustrate the examples of the compounds of Formula I.

Table I

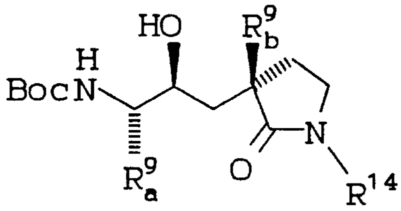

| $R^9_a$ | $R^9_b$ | $R^{14}$ |
|---|---|---|
| $CH_2Ph$ | $CH_2Ph$ | 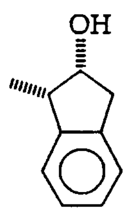 |
| $CH_2Ph$ | 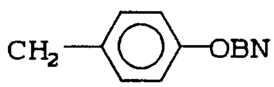 | 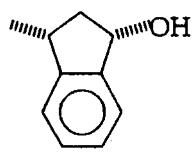 |
| $CH_2Ph$ | 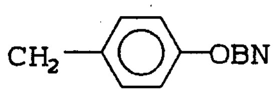 | 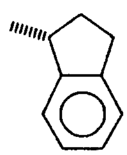 |
| $CH_2Ph$ | 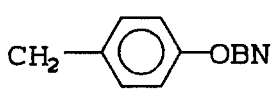 | 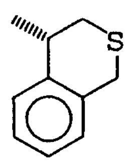 |
| $CH_2Ph$ | 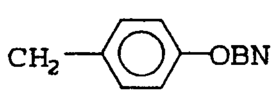 | 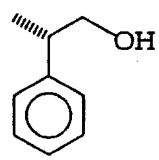 |

| $R^9_a$ | $R^9_b$ | $R^{14}$ |
|---|---|---|
| $CH_2Ph$ | 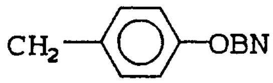 | 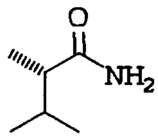 |
| $CH_2Ph$ | $CH_2Ph$ | 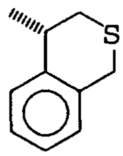 |
| $CH_2Ph$ | $CH_2Ph$ | 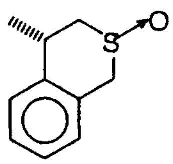 |
| $CH_2Ph$ | $CH_2Ph$ | 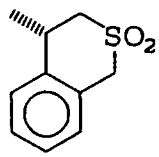 |
| 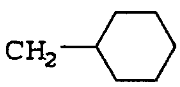 | 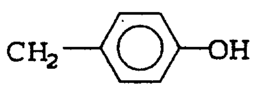 | 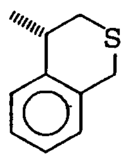 |

| $R^9_a$ | $R^9_b$ | $R^{14}$ |
|---|---|---|
| 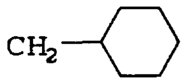 | $CH_2Ph$ | 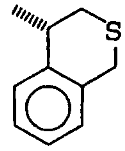 |
| 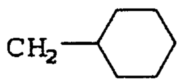 | 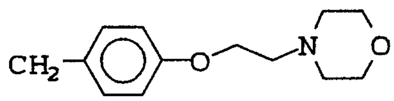 | 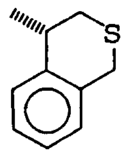 |
| 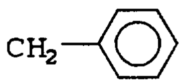 | 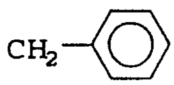 | 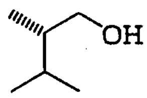 |
| $CH_2Ph$ | $CH_2Ph$ | 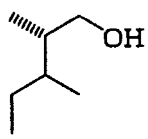 |
| 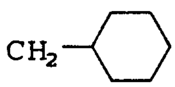 | $CH_2Ph$ | 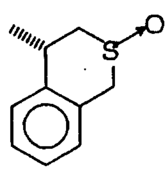 |

| $R^9_a$ | $R^9_b$ | $R^{14}$ |
|---|---|---|
| $CH_2Ph$ | $CH_2Ph$ | 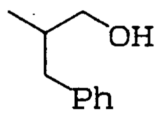 |
| $CH_2Ph$ | $CH_2Ph$ | 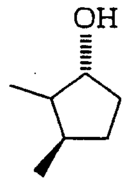 |
| $CH_2Ph$ | $CH_2Ph$ | 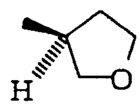 |
| $CH_2Ph$ | $CH_2Ph$ | 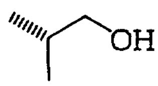 |
| $CH_2Ph$ | $CH_2Ph$ | 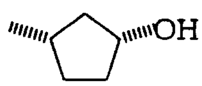 |

| $R^9_a$ | $R^9_b$ | $R^{14}$ |
|---|---|---|
| 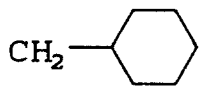 | 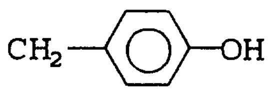 | 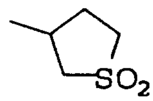 |
| $CH_2Ph$ | $CH_2Ph$ | 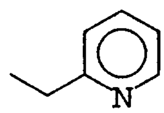 |
| 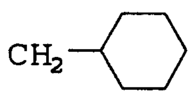 | 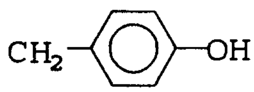 | 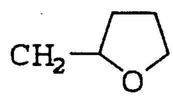 |
| $CH_2Ph$ | $CH_2Ph$ | 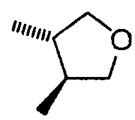 |
| 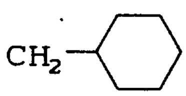 | 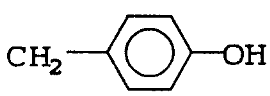 | 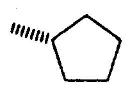 |

| $R^9_a$ | $R^9_b$ | $R^{14}$ |
|---|---|---|
| 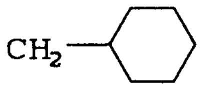 | 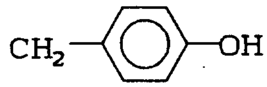 | 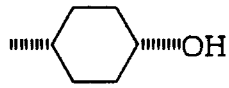 |
| $CH_2Ph$ | $CH_2Ph$ | 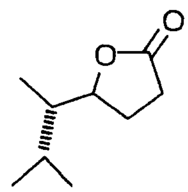 |
| 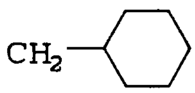 | 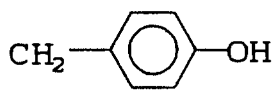 | 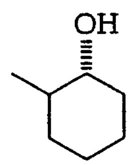 |
| 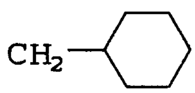 | 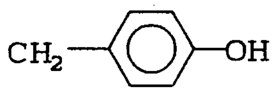 | 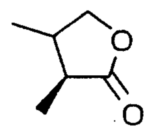 |
| 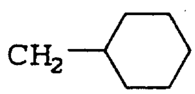 | 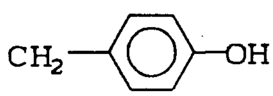 | 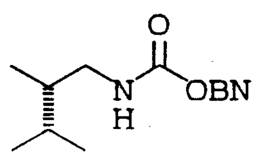 |
| 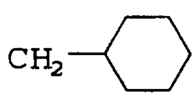 | $CH_2Ph$ | 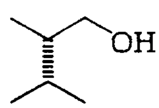 |

## Table II

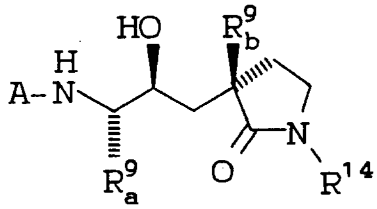

| A | $R^9_a$ | $R^9_b$ | $R^{14}$ |
|---|---|---|---|
| 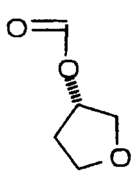 | $CH_2Ph$ | $CH_2Ph$ | 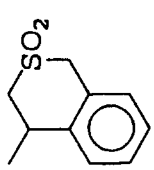 |
| 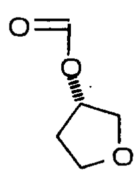 | $CH_2Ph$ | $CH_2Ph$ | 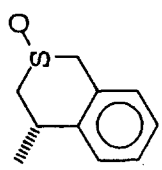 |
| 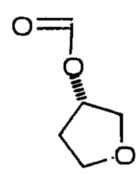 | $CH_2Ph$ | $CH_2Ph$ | 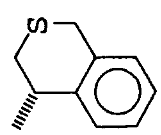 |
| 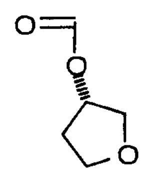 | 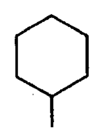 $CH_2$ | $CH_2Ph$ | 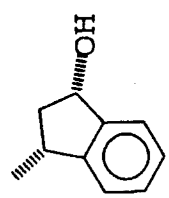 |

| A | $R^9_a$ | $R^9_b$ | $R^{14}$ |
|---|---|---|---|
| 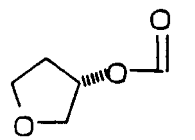 | 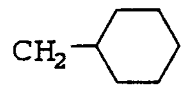 | 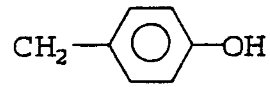 | 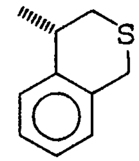 |
| 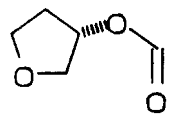 | 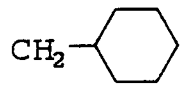 | 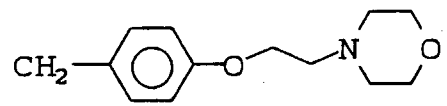 | 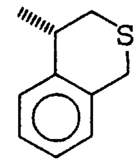 |
| 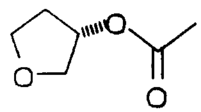 | 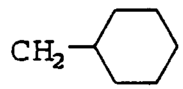 | 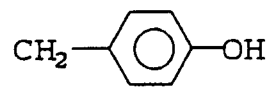 | 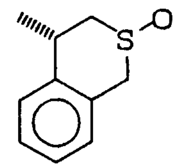 |
| 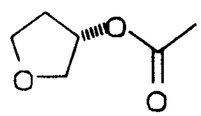 | 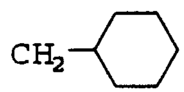 | 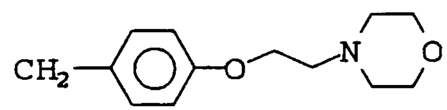 | 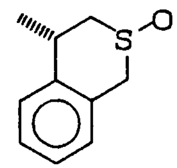 |

| A | $R^9_a$ | $R^9_b$ | $R^{14}$ |
|---|---|---|---|
| 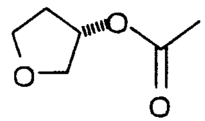 | $CH_2Ph$ | $CH_2Ph$ | 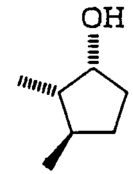 |
| 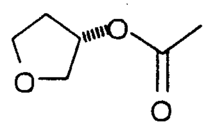 | $CH_2Ph$ | $CH_2Ph$ | 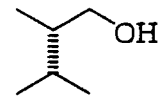 |
| 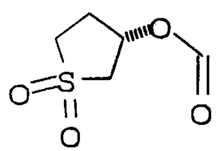 | $CH_2Ph$ | $CH_2Ph$ | 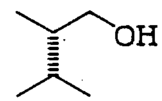 |
| 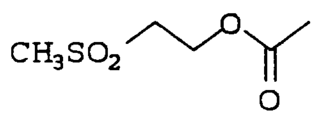 | $CH_2Ph$ | $CH_2Ph$ | 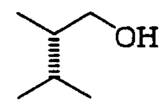 |

| A | $R^9_a$ | $R^9_b$ | $R^{14}$ |
|---|---|---|---|
| 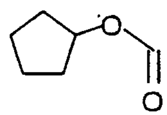 | $CH_2Ph$ | $CH_2Ph$ | 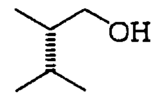 |
| 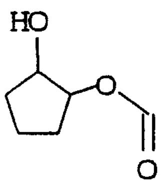 | $CH_2Ph$ | $CH_2Ph$ | 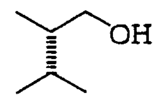 |
| 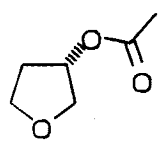 | $CH_2Ph$ | $CH_2Ph$ | 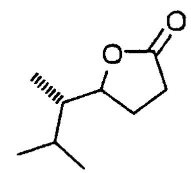 |
| 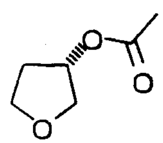 | $CH_2Ph$ | $CH_2Ph$ | 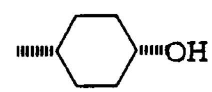 |

| A | $R^9_a$ | $R^9_b$ | $R^{14}$ |
|---|---|---|---|
| 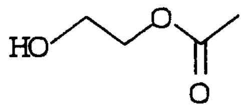 | $CH_2Ph$ | $CH_2Ph$ | 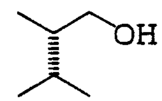 |
| 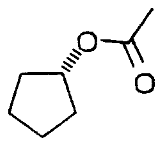 | $CH_2Ph$ | $CH_2Ph$ | 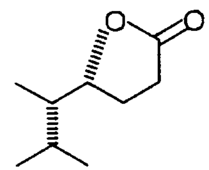 |
| 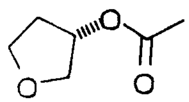 | $CH_2$ 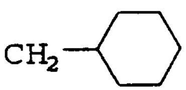 | $CH_2$ 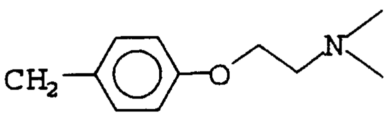 | 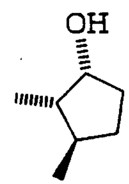 |
| 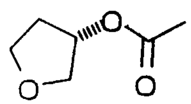 | $CH_2$ 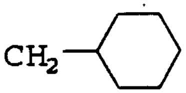 | $CH_2$ 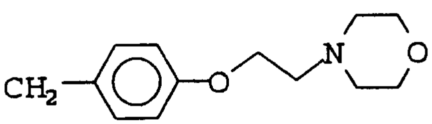 | 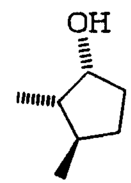 |

| A | $R^9_a$ | $R^9_b$ | $R^{14}$ |
|---|---|---|---|
| 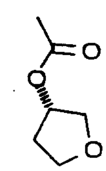 | 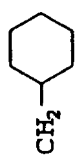 | 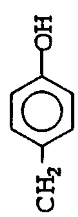 | 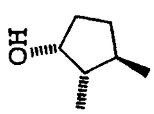 |
| 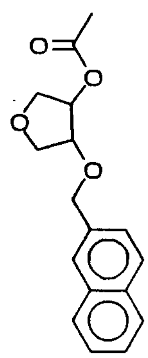 | 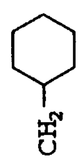 | 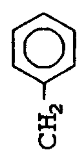 | 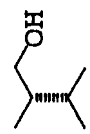 |
| 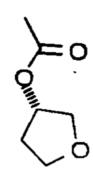 | 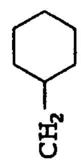 | 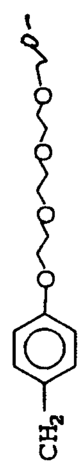 | 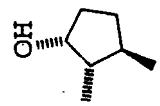 |

# Table III

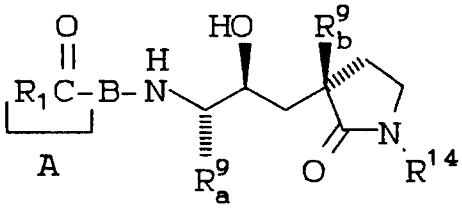

| $R_1$ | B | $R^9_a$ | $R^9_b$ | $R^{14}$ |
|---|---|---|---|---|
| 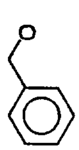 | 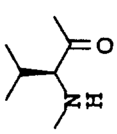 | $CH_2Ph$ | $CH_2Ph$ | 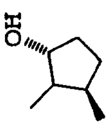 |
| 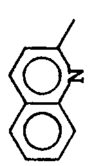 | 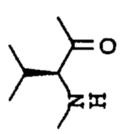 | $CH_2Ph$ | $CH_2Ph$ | 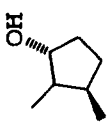 |
| 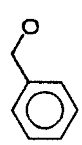 | 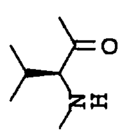 | 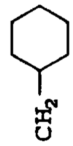 | 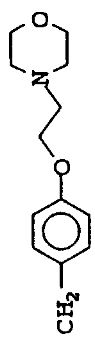 | 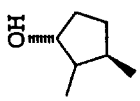 |
| 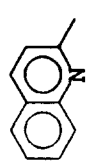 | 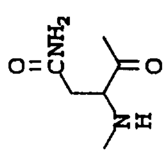 | 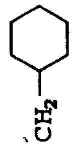 | 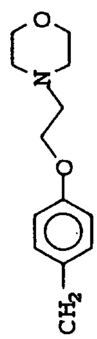 | 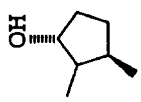 |
| 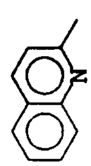 | 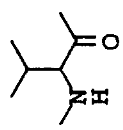 | 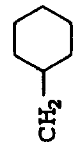 | 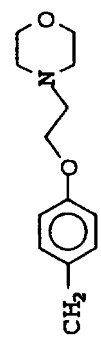 | 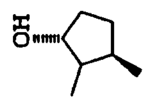 |

R$_1$ | B | $R^9_a$ | $R^9_b$ | $R^{14}$

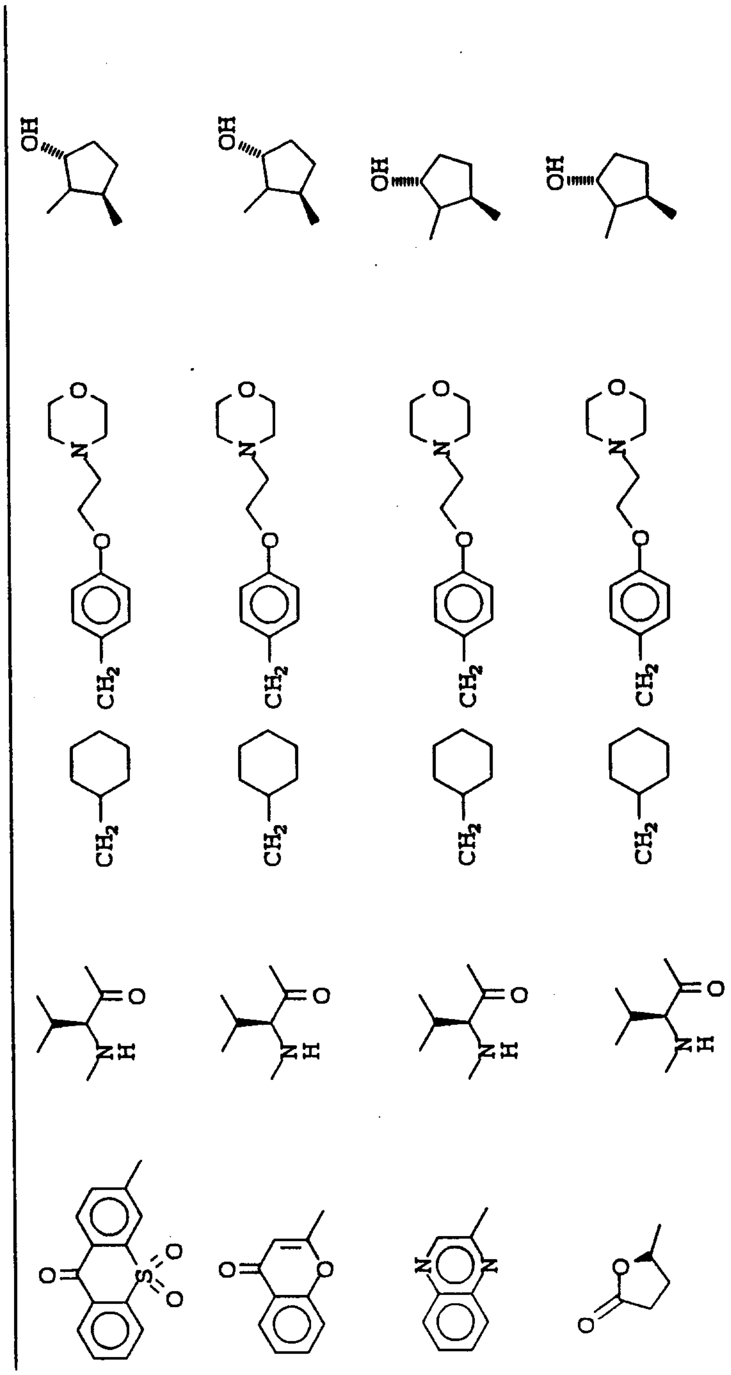

The compounds of the present invention are useful in the inhibition of HIV protease, the prevention or treatment of infection by the human immunodeficiency virus (HIV) and the treatment of consequent pathological conditions such as AIDS. Treating AIDS or preventing or treating infection by HIV is defined as including, but not limited to, treating a wide range of states of HIV infection: AIDS, ARC (AIDS related complex), both symptomatic and asymtomatic, and actual or potential exposure to HIV. For example, the compounds of this invention are useful in treating infection by HIV after suspected past exposure to HIV by e.g., blood transfusion, exchange of body fluids, bites, accidental needle stick, or exposure to patient blood during surgery.

In the present invention, compounds with asymmetric centers may occur as racemates, racemic mixtures and as individual diastereomers, with all isomeric forms of the compounds being included in the present invention.

For these purposes, the compounds of the present invention may be administered orally, parenterally (including subcutaneous injections, intravenous, intramuscular, intrasternal injection or infusion techniques),

by inhalation spray, or rectally, in dosage unit formulations containing conventional non-toxic pharmaceutically-acceptable carriers, adjuvants and vehicles.

Thus, in accordance with the present invention there is further provided a method of treating and a pharmaceutical composition for treating HIV infection and AIDS. The treatment involves administering to a patient in need of such treatment a pharmaceutical composition comprising a pharmaceutical carrier and a therapeutically-effective amount of a compound of the present invention, or a pharmaceutically-acceptable salt thereof.

These pharmaceutical compositions may be in the form of orally-administrable suspensions or tablets; nasal sprays; sterile injectable preparations, for example, as sterile injectable aqueous or oleagenous suspensions or suppositories.

When administered orally as a suspension, these compositions are prepared according to techniques well-known in the art of pharmaceutical formulation and may contain microcrystalline cellulose for imparting bulk, alginic acid or sodium alginate as a suspending agent, methylcellulose as a viscosity enhancer, and sweetners/flavoring agents known in the art. As immediate release tablets, these compositions may contain microcrystalline cellulose, dicalcium phosphate, starch, magnesium stearate and lactose and/or other excipients, binders, extenders, disintegrants, diluents and lubricants known in the art.

When administered by nasal aerosol or inhalation, these compositions are prepared according to techniques well-known in the art of pharmaceutical formulation and may be prepared as solutions in saline, employing benzyl alcohol or other suitable preservatives, absorption promoters to enhance bioavailability, flourocarbons, and/or other solubilizing or dispersing agents known in the art.

The injectable solutions or suspensions may be formulated according to known art, using suitable non-toxic, parenterally-acceptable diluents or solvents, such as mannitol, 1,3-butanediol, water, Ringer's solution or isotonic sodium chloride solution, or suitable dispersing or wetting and suspending agents, such as sterile, bland, fixed oils, including synthetic mono- or diglycerides, and fatty acids, including oleic acid.

When rectally administered in the form of suppositories, these compositions may be prepared by mixing the drug with a suitable non-irritating excipient, such as cocoa butter, synthetic glyceride esters or polyethylene glycols, which are solid at ordinary temperatures, but liquidify and/or dissolve in the rectal cavity to release the drug.

Dosage levels of the order of 0.02 to 5.0 or 10.0 grams-per-day are useful in the treatment or prevention of the above-indicated conditions, with oral doses two-to-five times higher. For example, infection by HIV is effectively treated by the administration of from 10 to 50 milligrams of the compound per kilogram of body weight from one to three times per day. It will be understood, however, that the specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

The present invention is also directed to combinations of the HIV protease-inhibitory compounds with one or more agents useful in the treatment of AIDS.

For example, the compounds of this invention can be given in combination with the antivirals, immunomodulaters, antibiotics or vaccines or other derivative forms thereof as listed in the following Table [source: Marketletter, Nov. 30. 1987, pp. 26-27; Genetic Engineering News, Jan. 1988, Vol. 8, 23]:

TABLE[1]

A. Antivirals

| Drug Name | Manufacturer | Indication |
|---|---|---|
| AL-721 | Ethigen | ARC, PGL |
| BETASERON (interferon beta) | Triton Biosciences | AIDS, ARC, KS |
| CARRISYN (polymannoacetate) | Carrington Labs | ARC |
| CYTOVENE (ganciclovir) | Syntex | CMV |
| DDC (dideoxycytidine) | Hoffmann-La Roche | AIDS, ARC |

[1]Abbreviations: AIDS (Acquired Immune Deficiency Syndrome); ARC (AIDS related complex); CMV (Cytomegalovirus, which causes an opportunistic infection resulting in blindness or death in AIDS patients); HIV (Human Immunodeficiency Virus, previously known as LAV, HTLV-III or ARV); KS (Kaposi's sarcoma); PCP (Pneumonocystis carinii pneumonia, an opportunistic infection); PGL (persistent generalized lymphadenopathy).

| Drug Name | Manufacturer | Indication |
|---|---|---|
| FOSCARNET (trisodium phosphonoformate) | Astra AB | HIV inf, CMV retinitis |
| HPA-23 | Rhone-Poulenc Sante | HIV infection |
| ORNIDYL (eflornithine) | Merrell Dow | PCP |
| PEPTIDE T (octapeptide sequence) | Peninsula Labs | AIDS |
| RETICULOSE (nucleophospho-protein) | Advanced Viral Research | AIDS, ARC |
| RETROVIR advanced (zidovudine; AZT) | Burroughs Wellcome | AIDS, ARC pediatric AIDS, KS, asympt HIV, less severe HIV, neurological involvement. |

| Drug Name | Manufacturer | Indication |
|---|---|---|
| RIFABUTIN (ansamycin LM 427) | Adria Labs | ARC |
| (trimetrexate) | Warner-Lambert | PCP |
| UA001 | Ueno Fine Chem Industry | AIDS, ARC |
| VIRAZOLE (ribavirin) | Viratek/ICN | AIDS, ARC, KS |
| WELLFERON (alfa interferon) | Burroughs Wellcome | KS, HIV, in comb with RETROVIR |
| ZOVIRAX (acyclovir) | Burroughs Wellcome | AIDS, ARC, in comb with RETROVIR |

B. Immunomodulators

| Drug Name | Manufacturer | Indication |
|---|---|---|
| ABPP KS (bropirimine) | Upjohn | Advanced AIDS, |
| AMPLIGEN (mismatched RNA) | DuPont HEM Research | ARC, PGL |

| Drug Name | Manufacturer | Indication |
| --- | --- | --- |
| (Anti-human alpha interferon antibody) | Advanced Biotherapy Concepts | AIDS, ARC, KS |
| Colony Stimulating Factor (GM-CSF) | Sandoz Genetics Institute | AIDS, ARC, HIV, KS |
| CL246,738 (CL246,738) | American Cynamid | AIDS |
| IMREG-1 | Imreg | AIDS, ARC, PGL, KS |
| IMREG-2 | Imreg | AIDS, ARC, PGL, KS |
| IMUTHIOL (diethyl dithio carbamate) | Merieux Institute | AIDS, ARC |

| Drug Name | Manufacturer | Indication |
| --- | --- | --- |
| IL-2 (interleukin-2) | Cetus | AIDS, KS |
| IL-2 (interleukin-2) | Hoffmann-La Roche Immunex | AIDS, KS |
| INTRON-A (interferon alfa) | Schering-Plough | KS |

| | | |
|---|---|---|
| ISOPRINOSINE (inosine pranobex) | Newport Pharmaceuticals | ARC, PGL, HIV seropositive patients |
| (methionine enkephalin) | TNI Pharmaceuticals | AIDS, ARC |
| MTP-PE (muramyl-tripeptide) | Ciba-Geigy | KS |
| THYMOPENTIN (TP-5) (thymic compound) | Ortho Pharmaceuticals | HIV infection |
| ROFERON (interferon alfa) | Hoffmann-La Roche | KS |
| (recombinant erythropoietin) | Ortho Pharmaceuticals | severe anemia assoc with AIDS & RETROVIR therapy |
| TREXAN (naltrexone) | DuPont | AIDS, ARC |
| TNF (tumor necrosis factor) | Genentech | ARC, in combination interferon gamma |

## C. Antibiotics

| Drug Name | Manufacturer | Indication |
|---|---|---|
| PENTAM 300 (pentamidine isethionate) | LyphoMed | PCP |

## D. Vaccines

Any one of a variety of AIDS or HIV vaccines presently under study and development can be used in combination with the compounds of this invention or salt or derivative forms thereof, in the treatment or prevention of AIDS and diseases of similar character caused by HIV.

It will be understood that the scope of combinations of the compounds of this invention with AIDS antivirals, immunomodulators, antibiotics or vaccines is not limited to the list in the above Table, but includes in principle any combination with any pharmaceutical composition useful for the treatment of AIDS.

EXAMPLE 1

Preparation of 4-(t-Butyldimethylsilyl)-oxy-benzyliodide

Step A: Ethyl-4-(t-butyldimethylsilyl)-oxy-benzoate

A quantity of 6.65 g of ethyl 4- hydroxy benzoate (40 mm, 1 eq.) was added to a round-bottom flask along with 60 mL dry $CH_2Cl_2$ and brought down to 0°C. A quantity of 6.63 g of t-butyldimethylsilyl chloride (44 mmoles, 1.1 eq.) and 6.06 g imidizole (89 mmoles, 2.2 eq.) were added to the reaction mixture at 0°C and the mixture allowed to stir under Ar overnight at 0°C. The reaction mixture was diluted with 150 mL $Et_2O$. The organic layer was washed with 10% HC1, saturated $NaHCO_3$, $H_2O$ and brine. The organic layer was dried over $MgSO_4$, filtered and stripped to yield 11.41 g of a pale yellow oil.

Step B: Ethyl-4-(t-butyldimethylsilyl)-oxy-benzyl alcohol

The product of Step A (11.41 g, 41 mmoles, 1 eq.) and 80 mL of fresh $Et_2O$ were placed in a round-bottom flask and were brought to -78°C. A volume of 62 mL of 1M $LiAlH_4$ in $EtO_2$ (6.2 mmoles, 1.5 eq.) was added and the mixture was allowed to stir at -78°C for 0.5 hours, brought up to 0°C for 1 hour, and quenched by adding successively dropwise to the reaction mixture 2.35 mL $H_2O$, 2.35 mL 15% NaOH, 7.05 mL $H_2O$. The resultant white precipatate was filtered off and washed with 400 mL $Et_2O$. The $Et_2O$ was concentrated to yield 9.56 g pale yellow oil.

Step C: 4-(t-butyldimethylsilyl)-oxy-benzylbromide

The product of Step B (4.13 g, 17.40 mm, 1 eq.) was dissolved in 60 mL fresh dry $Et_2O$ and was transferred to a flame-dried 300 mL round-bottom flask. A quantity of 10.75 g lithium bromide (8.66 mmoles, 0.50 eq.) and 275 mL 2,4,6-collidine (20.78 mmoles, 1.2 eq.) were added at room temperature. The reaction mixture was cooled to -78°C and 1.81 mL $PBr_3$ (19.05 mmoles, 1.1 eq.) was added to the flask via syringe. The reaction was allowed to warm to 0°C and stirred under Ar for 2 hours. The reaction was quenched by adding 60 mL $NaHCO_3$ (saturated). Organic and aqueous layers were separated and the aqueous layer was

extracted $Et_2O$. The organic layers were combined and washed twice with brine. Organic layer was dried over $MgSO_4$, filtered and stripped to yield 7.16 g crude product. Chromatography, using hexane and ethyl acetate as eluent, afforded the title compound as pure product.

Step D: 4-(t-butyldimethylsilyl)-oxy-benzyliodide

The product of Step C (4.01 g, 13.08 mm, 1 eq.) was placed in a round-bottom flask with stirrer. A volume of 75 mL acetone was added to the reaction flask along with 3.91 g NaI (26.08 mmoles 1.99 eq.). The reaction mixture was allowed to stir under Ar with light protection over 2 days. The reaction mixture was then diluted with 35 mL acetone and the insolubles filtered off and washed with acetone. The acetone was concentrated in vacuo to yield a green oily solid. The residue was taken up in 100 Ml $Et_2O$ and the organic layer washed with $H_2O$, 10% $NaHSO_3$, and $H_2O$. THe organic layer was dried over $MgSO_4$, filtered and stripped to yield 4.12 g title compound as product, which was stored in the freezer under light protection.

EXAMPLE 2

Preparation of N'-(4R,S)-(3,4-dihydro-1H-2-benzothiopyranyl)-3(S)-[3(S)-3(S)-tetrahydrofuranyloxycarbonylamino)-2(S)-hydroxy-4-cyclohexyl-butyl]-3(S)-(4-hydroxy-phenylmethyl)pyrrolidin-2-one

Step A: (3R,5S,1'S)-3-Allyl-5-[1-(1,1-dimethylethoxycarbonylamino)-2-cyclohexylethyl]-dihydrofuran-2(3H)-one

To a flame-dried round-bottom flask with stirrer was added 50 mL dry THF and brought to -78°C. A volume of 4.73 mL diisopropyl amine (33.73 mmoles of 2.1 eq.) was added to the flask via syringe, followed by 15.03 mL of 2.19 M n-BuLi in Hexane (32.90 mmoles, 2.05 eq.). The lithium diisopropyl amide was allowed to generate at -78°C for 2 hours.

A quantity of 5 g of (5S,1'S) -5-[1-(1,1-dimethylethoxycarbonylamino)-2-cyclohexylethyl]-dihydrofuran-2-(3H)-one (16.05 mmoles, 1 eq.) in 35 mL dry THF was added to the reaction mixture over 30 min at -78°C and the reaction mixture allowed to stir for 1 hour. A volume of 1.457 mL allyl bromide (16.85 mL, 1.05 eq.) was added to the flask at -78°C and the reaction mixture was warmed to -50°C and stirred for 1 hour. The reaction was quenched with 10 mL 1:1 glacial acetic acid:$H_2O$ and stirred overnight under Ar.

The reaction mixture was diluted with 250 mL $Et_2O$ and the organic layer was washed with $NaHCO_3$ saturated, 10% HCl, $NaHCO_3$ saturated, $H_2O$ and brine. The organic layer was dried over $MgSO_4$, filtered and stripped to yield 6.59 g crude product. The crude mixture was chromatographed using 85:15 hexane:ethyl acetate as eluent to yield title compound as pure product.

Step B: (3R,5S,1'S)-3-Allyl-((4-t-butyl-dimethylsilyl)oxy)-phenylmethyl-5-(1-((1,1-dimethylethoxycarbonylamino)-2-cyclohexylethyl)-dihydrofuran-2(3H)-one

To a flame-dried round-bottom flask with stirrer was added 30 mL dry THF and brought to -78°C. A volume of 2.41 mL diisopropyl amine (17.16 mmoles, 2.1 eq.) was added to the flask via syringe, followed by 7.65 mL in BuLi (16.75 mmoles, 2.05 eq.); the lithium diisopropylamide was allowed to generate for 1.5 hours at -78°C.

The product of Step A (2.87 g, 8.16 mm, 1 eq.) was added to the reaction mixture at -78°C over 30 min and the reaction mixture allowed to stir another 1 hour at -78°C.

The product of Example 1 (2.99 g, 8.61 mmoles, 1.06 eq.) was added at -78°C and then the reaction mixture was then warmed to -50°C and stirred at -50°C for 1 hour. The reaction was quenched by adding 10 mL 1:1 glacial acetic acid; $H_2O$ to the flask, resulting in a yellow product. The reaction mixture was stirred overnight under Ar.

The reaction mixture was diluted with 200 mL $Et_2O$ and the organic layer was washed with $NaHCO_3$ - (saturated), 10% HCl, $NaHCO_3$ (saturated), $H_2O$, and brine. The organic layer was dried over $MgSO_4$, filtered and stripped to yield 5.63 crude product. Crude material was chromatographed using 90:10 hexane:ethyl acetate as the eluent to yield the title compound as pure product.

Step C: (3R,5S,1'S)-3-Ethanal-3-((4-t-butyl-dimethylsilyl)oxy)phenylmethyl-5-(1-((1,1-dimethylethoxycarbonylamino)-2-cyclohexylethyl)-dihydrofuran-2(3H)-one

To a stirred solution of the product of Step B (2.24 g) in approximately 100 mL MeOH at -78°C was added $O_3$ (g) until the reaction mixture turned light blue. The reaction mixture was then purged with Ar and warmed to room temperature. About 1 mL dimethyl sulfide was added and the mixture stirred overnight. After concentration in vacuo, the reaction mixture was stripped from toluene and pumped on a high vacuum to afford a yellow foam as product.

Step D: N'-(4R,S)-(3,4-dihydro-1H-2-benzothiopyranyl) -3(S)-[3(S)-(1,1-dimethylethoxycarbonylamino)-2(S)-hydroxy-4-cyclohexyl-butyl]-3(S)-[((t-butyl-dimethylsilyl)oxy)phenylmethyl]-pyrrolidin-2-one

The product of Step C (1.65 g, 2.87 mmole, 1 eq.), (4R,S)-amino-3,4-dihydro-1H-2-benzothiopyran (0.52 g, 3.16 mmol, 1.1 eq.), MeOH, and 4Å crushed, oven-dried molecular series were added to a flame-dried flask. The reaction was stirred under Ar for 0.5 hours and 0.27 g (4.31 mmols, 1.5 eq.) sodium cyanoborohydride was added to the reaction mixture, followed by 4 drops glacial acetic acid. The mixture was stirred at room temperature under Ar overnight. A volume of 18 mL of 1N HCl was added to the mixture and stirring continued about 2 hours. The mixture was filtered and the white precipitate was washed with ethyl acetate. The aqueous and organic layers were separated and the aqueous layer was extracted with ethyl acetate. The ethyl acetate layers were combined, dried over $MgSO_4$, filtered, and concentrated to yield crude brown product. The crude material was chromatographed to obtain a pale yellow oil as product.

Step E: N'-(4R,S)-(3,4-dihydro-1H-2-benzothiopyranyl) -3(S)-[3(S)-(3(S)-tetrahydrofuranyloxycarbonylamino)-2(S)-hydroxy-4-cyc1ohexyl-butyl]-3(S)-(4-(t-butyldimethylsilyloxy)phenylmethyl)pyrrolidin-2-one

The product of Step D (1.83 mmole, 1 eq.) was dissolved in 15 mL $CH_2Cl_2$ under Ar and cooled to 0°C. A volume of 3 mL of trifluoroacetic acid was added. The reaction mixture was allowed to warm to room temperature and stir under Ar overnight. The mixture was concentrated in vacuo to yield a brown residue which was taken up in 100 mL ethyl acetate, then washed with saturated $NaHCO_3$ and brine. The organic layer was dried over $MgSO_4$, filtered, and concentrated to yield 1.03 g of a brown oil. Subsequent mixing with 3(S)-tetrahydrofuranyl succinimidyl carbonate (0.44 g, 1.91 mmole, 1.16 eq.), and 20 mL $CH_2Cl_2$ was carried out, followed by stirring under Ar at room temperature. A volume of 344 $\mu$L (0.25g. 2.48 mmoles, 1.5 eq.) triethyl amine was added and the reaction stirred for 2 days under Ar. The reaction mixture was diluted with 100 mL $CHCl_3$ and the orgainc layer washed with saturated $NaHCO_3$ and brine. The organic layer was dried over $MgSO_4$, filtered and concentrated to yield a fluffy solid upon pumping on high vac. The crude material was chromatographed using 5:1 hexane:ethyl acetate as the eluent to afford the title compound.

Step F: N'-(4R,S)-(3,4-dihydro-1H-2-benzothiopyranyl) -3(S)-[3(S)-(3(S)-tetrahydrofuranyloxycarbonylamino)-2(S)-hydroxy-4-cyclohexylbutyl]-3(S)-(4-hydroxyphenylmethyl)-pyrrolidin-2-one

A volume of 5.00 mL of 1.0M tetrabutylammnonium fluoride solution in THF (5.00 mmoles, 5 eq.) was added to a flask containing 0.74 g (1.00 mmoles, 1 eq.) of the product of Step E. The mixture was stirred under Ar overnight and then the reaction was concentrated in vacuo and taken up in 100 mL ethyl acetate, then washed with 10% HCl, $H_2O$, saturated $NaHCO_3$, $H_2O$ and brine. The organic layer was dried over $MgSO_4$, filtered, and concentrated to yield 0.73 g of a crude tan solid product. The crude product was chromatographed using 3:97 ispropyl alcohol:$CHCl_3$ as the eluent to obtains the title compound as a white foamy solid.

EXAMPLE 3

Preparation of N'-(4S)-(3,4-dihydro-1H-2-benzothiopyranyl)-3(S)-[3(S)-[3(S)-tetrahydrofuranyloxycarbonylamino)-2(S)-hydroxy-4-cyclohexyl-butyl]-3(S)-((4-(2-(4-morpholino)ethoxy)phenyl)methyl)-pyrrolidin-2-one

Step A: Chloroethyl morpholine, free base

Chloroethyl morpholine hydrochloride (40 g FW = 186.08) was dissolved in 280 mL of a half saturated solution of $K_2CO_3$. A volume of 250 mL hexane was added to the flask and the mixture stirred. The hexane layer was separated off and the remaining aqueous layer extracted with hexane. The hexane washes were combined and washed with brine, dried over $Na_2SO_4$, filtered, and stripped to yield 24.11 g free base (a liquid).

Step B: N'-(4S)-(3,4-dihydro-1H-2-benzothiopyranyl)-3 (S)-[3(S)-(3(S)-tetrahydrofuranyloxycarbonylamino)-2-(S)-hydroxy-4-cyclohexyl-butyl]-3(S)-((4-(2-(4-morpholino)ethoxy)phenyl)methyl)-pyrrolidin-2-one

The quantity of 0.49 g of the product of Example 2 (0.78 mmole, 1 eq.) was dissolved in 8 mL 1,4-dioxane. A quantity of chloroethyl morpholine was added via pipet using 3 mL 1,4-dioxane for the transfer. A quantity of 2.54g (7.8 mmoles, 10 eq.) $Cs_2CO_3$ (s) was added to the flask along with 5 mL addition 1,4-dioxane. The reaction mixture was heated under Ar gas at 80°C with vigorous stirring for 3 hours. The mixture was then cooled to room temperature, filtered, and concentrated in vacuo. The crude mixture was pumped on a high vacuum overnight. The crude mixture was chromatographed (twice in 5:95 isopropyl alcohol:CH $Cl_3$ and 5:95 MeOH: CH $Cl_3$) to obtain product.

| Analysis calc for $C_{41}H_{57}N_3O_7S$: | | | |
|---|---|---|---|
| | C, 64.27; | H, 7.48; | N, 5.44 |
| Found: | C, 64.33; | H, 7.36; | N, 5.60 |

EXAMPLE 4

Preparation of N'-[(4S),(2RS)-(3,4-dihydro-1H-2-benzooxothiopyranyl)]-3(S)-[3(S)-(3(S)-tetrahydrofuranyloxycarbonylamino)-2(S)-hydroxy-4-(cyclohexyl)-butyl]-3(S)-(4-hydroxyphenylmethyl)-pyrrolidin-2-one

To a stirred solution of the product of Example 2 (105 mg, 0.17 mmole, 1 eq.) in 11 mL MeOH, at room temperature, was added 364 mg $NaIO_4$ (1.7 mmoles, 10 eq.) and 10 mL $H_2O$. The reaction was stirred for 1 hour and concentrated in vacuo to yield a yellow white slurry. The reaction mixture was then dissolved in ethyl acetate and the aqueous layer separated off. The ethyl acetate layer was dried over $MgSO_4$, filtered, and stripped to yield 150 mg crude product. Crude material was purified by chromatography using 5:95 MeOH:$CHCl_3$ as the eluent to yield pure product after subsequent trituration with $Et_2O$.

| Analysis calc for $C_{35}H_{46}N_2O_7S$: | | | |
|---|---|---|---|
| | C, 63.83; | H, 7.38; | N, 4.25 |
| Found: | C, 63.84; | H, 7.11; | N, 4.35 |

EXAMPLE 5

Preparation of N'-(4S),(2RS)-(3,4-dihydro-1H-2-benzooxopyranyl)-3(S)-[3(S)-(3(S)-tetrahydrofurany-loxycarbonylamino)-2(S)-hydroxy-4-cyclohexyl-butyl]-3-(S)-((4-(2-(4-morpholino)ethoxy)phenyl)methyl)-pyrrolidin-2-one

The product of Example 3 is oxidized by the procedure of Example 4 to yield the title compound.

| Analysis calc for $C_{41}H_{57}N_3O_8S$: | | | |
|---|---|---|---|
| | C, 59.38; | H, 6.89; | N, 4.97 |
| Found: | C, 59.01; | H, 6.72; | N, 5.36 |

EXAMPLE 6

Preparation of N'-[(5S, 1'S)-(2-(methyl)ethyl-dihydrofuran-2-(3H)-one)yl]-3(S)-[3(S)-(1,1'-dimethylethoxy-carbonylamino)-3(S)-hydroxy-4-phenyl-butyl]-3(S)-phenylmethyl-pyrrolidin-2-one

The lactone, (5S, 1'S)-5-(1'-((1,1'-dimethylethoxycarbonyl)amino)-2-(methyl)-propyl-dihydrofuran-2-(3H)-one (0.059 g, 0.23 mmol) was dissolved in 10 mL ethyl acetate and placed in a 20 mL round-bottom flask containing a magnetic stirring bar. A stream of $N_2$ gas was passed over the solution while it was stirred and cooled to 0 ° C using an ice bath. HCl gas was then bubbled through the solution until saturated, while keeping the temperature below 5 ° C.
Stirring was continued for 10 min. The solvent was removed under reduced pressure to obtain a white powder, which was then redissolved in 10 mL MeOH and placed under a $N_2$ atmosphere. To this stirring mixture was added 1 drop glacial acetic acid and some 4A molecular sieves followed by the aldehyde, (3R, 55, 1'S)-3-ethanal-3-phenylmethyl-5-(1-((1,1'-dimethylethoxycarbonyl)amino)-2-phenylethyl-dihydrofuran-2-(3H)-one. Stirring was continued for 10 min followed by the addition of $NaCNBH_3$ (0.1 g, 0.23 mmol) after which it was stirred for 18 hrs. The reaction was quenched using 10 mL of a 10% citric acid solution and stirred for 30 min. The mixture was then diluted with water and extracted with 3 x 20 mL ethyl acetate. The organics were combined and washed with water, aqueous saturated $Na_2CO_3$ and brine. This solution was then dried over $Na_2SO_4$, filtered and the solvent removed. The crude reaction product was purified using Preparative Thin Layer Chromatography (5% $MeOH/CH_2Cl_2$) to obtain 0.023 g (0.04 mmol, 17%) of title compound.
M.P. = 78 - 80 ° C

EXAMPLE 7

Preparation of N'-[(5S, 1'S)-(2-(methyl)-propyldihydrofuran-2-(3H)-one)yl]-3(S)-[3(S)-(3S-(tetrahydrofuranoxycarbonylamino)-2(S)-hydroxy-4-phenylbutyl]-3(S)-phenylmethyl-pyrrolidin-2-one

The product of Example 6 (0.019 g, 0.033 mmol) was dissolved in 10 mL ethyl acetate and cooled to 0 ° C under a stream of $N_2$. The solution was treated with HCl gas and worked up as above to give the amine-HCl salt. This salt was dissolved in 10 mL $CH_2Cl_2$ and to it was added triethylamine (0.0066 g, 0.0092 mL, 0.066 mmol) followed by 3-(S)-tetra-hydrofuranyl succinimidyl carbonate (0.008 g, 0.033 mmol). The reaction was stirred for 4 hrs., poured into 10 mL 10% citric acid solution and extracted with $CH_2Cl_2$. The organics were combined and washed with citric acid, water, aqueous saturated $Na_2CO_3$ and brine. The solution was then dried ($Na_2SO_4$), filtered and the solvent removed. Purification by Preparative Thin Layer Chromatography (5% $MeOH/CH_2Cl_2$) yielded .016 g (0.026 mmol, 82%) of title compound.
M.P. = 72 - 74 ° C

EXAMPLE 8

Preparation of (5R, 1'S)-5-(1'-(1,1'-dimethylethoxy-carbonylamino)-2-(methyl)-propyl-dihydrofuran-2-(3H)-one

A quantity (0.03 g, 0.12 mmol) of (5S, 1'S)-5-(1'-((1,1'-dimethylethoxy-carbonyl)amino)-2-(methyl) - propyl-dihydrofuran-2-(3H)-one was placed in a 25 mL round-bottom flask containing a magnetic stirring bar and fitted with $N_2$ adaptor and dissolved in 5 mL methanol. The solution was cooled to -78 ° C in a dry ice/acetone bath. Dimethyl amine gas was condensed into the reaction vessel until the total volume was doubled. The vessel was stoppered and the reaction mixture stirred 18 hrs. The solvent was then removed to yield 0.036 g (0.12 mmol) of the alcohol, N,N-Dimethyl-4(S)-hydroxy-5(S)-(1,1'-dimethylethoxycarbonylamino)-6-methyl heptaneamide, which was used without further purification.

The alcohol was then dissolved in 0.2 mL dry pyridine in a 1 mL round-bottom flask fitted with a $N_2$ adaptor and a magnetic stirring bar. To this was added 0.016 g (0.011 mL, 0.14 mmol) methanesulfonyl chloride and the mixture stirred at room temperature for 48 hrs. The pyridine was then removed in vacuo and the dark residue partitioned between ethyl acetate and 10% citric acid. The layers were separated and the aqueous phase was extracted with ethyl acetate. The organics were combined and washed with 10% citric acid, water, aqueous saturated $Na_2CO_3$ and brine. The solution was then dried ($Na_2SO_4$), filtered and the solvent removed to afford .013 g (.051 mmol, 42%) of the title compound.

EXAMPLE 9

Preparation of N'[(5R, 1'S)-(2-(methyl)-propyldihydrofuran-2-(3H)-one)yl]-3(S)-[3(S)-(3(S)-(tetra-hydrofuranoxycarbonylamino)-2(S)-hydroxy-4-phenylbutyl]-3(S)-phenylmethyl-pyrrolidin-2-one

The product of Example 8 was dissolved in 5 mL ethyl acetate and treated with HCl gas as above to give the amine which was dissolved in 2 mL MeOH in a 5 mL round-bottom flask fitted with a $N_2$ adaptor and a magnetic stirring bar. To this mixture was added the aldehyde (3R, 5S, 1'S)-3-ethanal-3-phenylmethyl-5-(1-(3(S)-tetrahydrofuranoxy)carbonylamino)-2-phenylethyl)-dihydrofuran-2-(3H)-one (0.021 g, 0.05 mmol), 1 drop glacial acetic acid and some 4A molecular sieves. The reaction was stirred for 2 hr. Then $NaCNBH_3$ (0.0032 g, 0.055 mmol) was added and the reaction was stirred for an addition 18 hrs. The reaction was worked up as described in Example 8, and the resulting product was purified by Preparative Thin Layer Chromatography to yield 0.009 g (0.016 mmol, 32%) of title compound.

EXAMPLE 10

Preparation of N'-[2(S)-3-methyl-1-butanol]-3(S)-[3(S)-(3(S)-tetrahydrofuranoxycarbonyl amino)-2(S)-hydroxy-4-phenyl-butyl]-3(S)-phenylmethyl-pyrrolidin-2-one

Step 1: (3R, 5S, 1'S)-3-allyl-3-phenylmethyl-5-(1-((1,1'-dimethylethoxy-carbonyl)amino)-2-phenylethyl)-dihydrofuran-2-(3H)-one

In a flame-dried, three-neck, 500 mL round-bottom flask that was equipped with a digital thermometer, diisopropylamine (7.28 mL, 52 mmol) and freshly distilled THF (15 mL) were mixed under an Argon atmosphere. The solution was cooled to -78°C, and n-butyllithium (2.5 M, 20.4 mL, 51 mmol) was syringed into the flask. The heterogeneous mixture was warmed to 0°C at which point the solution was cooled back to -78°C and (3R, 5S, 1'S)-3-allyl-5-(1-((1,1'-dimethylethoxycarbonyl)amino)-2-phenylethyl) -dihydrofuran-2-(3H)-one in THF (15 mL) was added via double-ended needle maintaining the temperature below -65°C. The yellow solution was warmed to -45°C over 1 1/2 hour, then cooled back to -78°C. Benzyl bromide (5.8 mL, 48.6 mmol) was added dropwise, keeping the temperature at -78°C. The reaction was stirred at -78°C for 1 1/2 hours or until TLC (25% ethyl acetate/hexane) showed no starting material remaining. Aqueous citric acid (10%, 20 mL) was added, and the mixture was stirred for 15 minutes at room temperature. The mixture was poured into 100 mL ethyl acetate, and the layers were separated. The aqueous layer was washed with 20 mL ethyl acetate. The organic layers were combined, washed with $H_2O$ (20 mL), saturated $NaHCO_3$ (20 mL), brine, and dried over $MgSO_4$. The aqueous layers were re-extracted with ethyl acetate (20 mL), and the organic layers combined. The solvent was evaporated to give a viscous yellow oil which was chromatographed in 20% ethyl acetate/hexane to give 7.5 g (72% yield) pure product.

Step 2: (3R, 5S, 1'S)-3-ethanal-3-phenylmethyl-5-(1-((1,1'-dimethylethoxycarbonyl)amino)-2-phenylethyl)-dihydrofuran-2-(3H)-one

Lactone from step 1 (2.899 g, 6.85 mmol) was dissolved MeOH/$CH_2C1_2$ (100 mL, 4:1). The solution was cooled to -78°C and ozone was bubbled into the solution until a deep blue color persisted (10 minutes). After purging the flask of excess ozone, dimethyl sulfide (4 mL) was added, and the reaction was allowed to warm to room temperature and stirred for 48 hours. The solvents were evaporated, and the residue was chromatographed twice in 10% acetone/ $CHCl_3$ to give 1.95 g (65% yield) of desired aldehyde.

Step 3: N'-[2(S)-3-methyl-1-butanol]-3(S)-[3(S) (1,1-dimethylethoxycarbonylamino)-3(S)-hydroxy-4-phenyl-butyl]-3(S)-phenylmethylpyrrolidin-2-one

(3R, 5S, 1'S)-3-ethanal-3-phenylmethyl-5-(1-((1,1'-dimethylethoxycarbonyl)amino)-2-phenylethyl)-dihydrofuran-2-(3H)-one (1.388g, 3.18 mmol) prepared from Step 2 above was dissolved in MeOH (20 mL). Activated 3A molecular sieves (powder), and S(+)-2-amino-3-methyl-1-butanol (0.4276g, 3.65 mmol) dissolved in MeOH (5 mL) were added to the mixture and imine formation allowed to proceed for 2 hours at room temperature. $NaCNBH_3$ (0.319g, 5.08 mmol) was added to the flask, followed by 1 mL glacial acetic acid. The reaction was allowed to proceed overnight under Argon. Excess $NaCNBH_3$ was quenched by the addition of 10% citric acid, and the resulting slurry was stirred for 2 hours. The MeOH was evaporated, the residue that remained was taken up in ethyl acetate, washed with brine (40 mL) and 10% citric acid (30

mL). The aqueous layer was extracted with ethyl acetate (6 x 100 mL). Organic layers were combined, dried over $MgSO_4$, filtered, and concentrated. The residue was purified by flash chromatography in 5% $MeOH/CHCl_3$ to give 0.819 g (49% yield) of desired product; m.p.165-167°C; calc'd for $C_{31}H_{44}N_2O_5$: (524.7067):

| | | | |
|---|---|---|---|
| | C, 70.96; | H,8.45; | N,5.34; |
| Found: | C, 70.81; | H,8.40; | N, 5.27. |

Step 4: N'-[2(S)-3-methyl-1-butanol]-3(S)-[3(S)-(3(S)-tetrahydrofuranoxycarbonylamino)-2(S)-hydroxy-4-phenyl-butyl]-3(S)-phenylmethylpyrrolidin-2-one

The product from step 3 above (84mg, 0.16 mmol) was dissolved in 3 mL $CH_2Cl_2$ and cooled to 0°C. Trifluoroacetic acid (1.23 mL, 16mmol) was added and the reaction was stirred for 45 minutes at which time TLC indicated no starting material remained. The solvents were evaporated and the oily residue was dissolved in 1 mL $CHCl_3$ and azeotroped with toluene (20 mL). The residue was dissolved in 1 mL absolute EtOH, azeotroped with toluene (5mL), and dried under vacuum.

The TFA salt was dissolved in $CH_2Cl_2$ (3 mL) with 3(S)-tetrahydrofuranyl succinimidyl carbonate (40.4 mg, 0.176 mmol). Triethylamine (0.027 mL, 0.192 mmol) was added via syringe and the reaction was allowed to proceed with warming to room temperature overnight. The solvents were removed and the residue was purified by flash chromatography (12% acetone/$CHCl_3$). The product was further purified by recrystallizing in ethyl acetate/hexane to give 44 mg of desired product. m.p. 136-137°C

## EXAMPLE 11

Preparation of N'-[2(S)-3-methyl-1-butanol]-3(S)-[3(S)-(4(RS)-benzyloxy-3(RS)-tetrahydrofuranoxy-carbonyl-amino)-2(S)-hydroxy-4-phenyl-butyl]-3(S)-phenylmethyl-pyrrolidin-2-one

Step 1: 4(RS)-benzyloxy-3(RS)-tetrahydrofuranylsuccinimidyl

A 500 mL round-bottom flask was charged with 3,4-dihydroxy-tetrahydrofuran (18.73g 180 mmol), benzaldehyde (20 mLs) p-toluenesulfonic acid (0.5g) and toluene (200 mLs). The flask was equipped with a dean stark apparatus, reflux condensor and heated for 4 hours until water no longer collected in the dean-stark trap. The reaction was cooled to room temperature and washed with $Na(CO_3)_2$, $H_2O$ and saturated NaCl. After drying and removal of volatile material the yellow oil that remained was used in the next step without purification. In a 500 mL round-bottom flask the yellow oil from above was dissolved in $CH_2Cl_2$ cooled to -78°C and treated sequentially with $TiCl_4$ (180 mLs, 1M solution $CH_2Cl_2$) and triethyl silane (27 mLs). The reaction was stirred for 1 hr at that temperature, then warmed to 0°C over 1 hr and stirred for an additional 2 hours. The reaction was quenched by pouring into ice cold saturated $NaHCO_3$. The layers were separated and the organics dried ($MgSO_4$). The aqueous layer was re-extracted with $CH_2Cl_2$ (2 X 200 mLs), combined and washed with NaCl and pooled with initial organic extracts. Product was purified via flash LC yielding a colorless oil. 4(RS)-benzyloxy-3(RS)-hydroxytetrahydrofuran (2.88g) was dissolved in $CH_2Cl_2$ (20 mLs) and cooled to 0°C. A solution of $COCl_2$ (12.5% in toluene) was added via addition funnel over 5 minutes. The resulting solution was aged for 24 hours with gradual warming to room temperature. Argon was passed through the solution for 15 minutes followed by removal of all volatiles at water aspirator pressure. The crude oil was azeotroped with toluene (2 X 10 mL) and used without further purification. The crude chloroformate was dissolved in $CH_2Cl_2$ cooled to 0°C. To this cold solution was added N-hydroxysuccinimide (1.7g) and triethylamine (2.1 mL). The mixture was stirred for 12 hours at room temperature, then diluted with $CH_2Cl_2$ (50 mL) and washed with $NaHCO_3$, NaCl and dried ($Na_2SO_4$). Flash LC using ethyl acetate/hexane mixtures provided the desired compound in low yield contaminated with 4-(RS)-benzyloxy-3(RS)-hydroxytetrahydrofuran.

Step 2: N'-[2(S)-3-methyl-1-butanol]-3(S)-[3(S)-amino-2(S)-hydroxy-4-phenyl-butyl]-3(S)-phenylmethylmethyl-pyrrolidin-2-one hydrochloride

N'-[2(S)-3-methyl-1-butanol]-3(S)-[3(S)-(1,1-dimethylethoxycarbonyl amino)-2(S)-hydroxy-4-phenyl-butyl]-3(S)-phenylmethyl-pyrrolidin-2-one was dissolved in $CH_2Cl_2$ and cooled to 0°C. Anhydrous ether was

saturated with HCl gas, and an equal portion of the resulting solution was added to the $CH_2Cl_2$. The mixture was stirred for 2 hours or until TLC showed no starting material. The solvents were evaporated, and the residue was dried under vacuum.

Step 3: N'-[2(S)-3-methyl-1-butanol]-3(S)-[3(S)-(4-(RS)-benzyloxy-3(RS)-tetrahydrofuranoxycarbonylamino)-2(S)-hydroxy-4-phenyl-butyl]-3-(S)-phenylmethyl-pyrrolidinone

The hydrochloride salt (50 mg, 0.099mmol), obtained from Step 2, was dissolved in DMF (1 mL) with the cis-hydroxy furan carbonate (100 mg, 0.148 mmol) from step 1. The mixture was cooled to 0 ° C, and triethylamine (0.015 mL, 0.109 mmol) was added. The solvents were evaporated in vacuo after 4 hours and the residue was diluted with 30 mL ethyl acetate and washed with saturated $NaHCO_3$. The aqueous layer was extracted with ethyl acetate (2 x 30 mL). The organics were combined, washed with brine, and dried over $MgSO_4$. After filtering and concentrating, the oil was purified by flash chromatography in 5% isopropanol/$CHCl_3$ to yield 50 mg of the product (1:1 diastereomeric mixture).
m.p. 50-60 ° C

EXAMPLE 12

Preparation of N'-[2(S)-3-methyl-1-butanol]-3(S)-[3(S)-(4(RS)-hydroxy-3(RS)-tetrahydrofuranoxy-carbonylamino)-3(S)-hydroxy-4-phenyl-butyl]-3(S)-phenylmethylpyrrolidin-2-one

N'-[2(S)-3-methyl-1-butanol]-3(S)-[3(S)-[4(RS)-benzyloxy-3(RS)-tetrahydrofuranoxycarbonyl amino-3(S)-hydroxy-4-phenyl-butyl]-3(S)-phenylmethylpyrrolidin-2-one (mixture of diastereomers) was dissolved in 2 mL 95 % ethanol containing 10% Pd on carbon. The mixture was hydrogenated under a hydrogen-filled balloon overnight. The catalyst was filtered, and the filtrate was concentrated. The residue was purified by flash chromatography in 10% isopropanol/$CHCl_3$ a quantity of 22 mg of a white solid was collected and further purified by recrystallization using ether/hexanes to give 13 mg of final product.
m.p. 140-141 ° C

EXAMPLE 13

N'-(2(S)-cyclopentyl-1(R)-hydroxy-3(R)-methyl)-3(S)-[3(S)-(3(S)-tetrahydrofuranoxycarbonyl amino)-2(S)-hydroxy-4-phenyl-butyl]-3(S)-[4-(2-(4-morpholinyl)ethoxy)phenylmethyl]-pyrrolidin-2-one

Step 1: Preparation of Aldehyde

(3R, 5S, 1'S)-3-allyl-3-((4-t-butyl-dimethylsilyl)oxy)phenylmethyl-5-(1-((1,1'-dimethylethoxycarbonyl)-amino)-2-cyclohexylethyl)-dihydrofuran-2-(3H)-one was dissolved in MeOH/$CH_2Cl_2$ (10:1) and cooled to -78 ° C. Ozone was bubbled through this solution until a deep blue color was maintained. Argon gas was then bubbled through the solution to purge the excess ozone. At - 78 ° C, dimethyl sulfide (4 mL) was added and the solution was allowed to warm to room temperature. The solution was stirred for an additional 3 hours, then the solvents were removed and the residue was purified by flash LC (3:1 Hexane: EtOAc) to give 3.43 g of product.

Step 2: Preparation of N'-[2(S)-cyclopentyl-1(R)-hydroxy-3(R)-methyl]-3(S)-[3(S)-(1,1-dimethylethoxycarbonylamino)-2(S)-hydroxy-4-phenyl-butyl]-3(S)-[4-(hydroxy)phenyl-methyl]-pyrrolidin-2-one

The aldehyde from step 1 above (0:711 g, 1.24mmol), 2-amin 3-methyl-cyclopentanol HCl (0.208 g, 1.37 mmol) and activated 3A moleculer sieves (powder) were placed in a flame-dried 25 mL round-bottom flask equipped with stir bar. A volume of MeOH (3 mL) was added and this mixture stirred for 0.5 hours. A quantity of $NaCNBH_3$ (0.233g, 3.72mmol) was added to the solution followed by glacial acetic acid (10 eq.) and the reaction was stirred overnight. After 14 hours HPLC indicated a small amount of starting aldehyde was present so an additional 20 mgs of the amine HCl was added and the reaction stirred for 2 more hours. The solution was filtered and the filtrate treated with 10% citric acid (4 mL). The MeOH was then removed via rotoevaporator and the solution was made basic with N NaOH. The mixture was extracted with $CHCl_3$ (3 x 30 mL), combined and dried over $MgSO_4$. Following removal of all volatiles the residue (800 mg) that remained was dissolved in toluene (6 mL) and exposed to 1-hydroxy benzotriazole (50 mg). This solution was heated to 80 ° C overnight. Upon cooling to room temperature the reaction was concentrated and the

residual oil was dissolved in THF/INHCl (4:1) and stirred for 72 hours. The reaction was neutralized with solid $NaHCO_3$ (60 mg) and concentrated. The aqueous residue was extracted with EtOAc (3 x 40 mL). The organic layers were collected and dried ($MgSO_4$). The crude product was purified by flash LC (1:1 EtOAc: $CH_2Cl_2$) to give 540 mg of the desired phenol.

Step 3: N'-(2(S)-cyclopentyl-1(R)-hydroxy-3(R)-methyl)-3(S)-[3(S)-(1,1-dimethylethoxycarbonylamino)-2(S)-hydroxy-4-phenyl-butyl]-3(S)-[4 -(2-(4-morpholinyl)ethoxy)-phenylmethyl]-pyrrolidin-2-one

The phenol from step 2 (0.350 g, 627 mmol), chloroethyl morpholine(0.468 g, 3.13 mmol) and $Cs_2CO_3$ - (0.68g, 2.089mmol) were placed in a 50 mL round-bottom flask and dissolved in dry dioxane (6 mL). This solution was stirred at 80 ° C overnight (with condenser) under an Argon atmosphere. After 14 hours it was cooled to room temperature, diluted with $CHCl_3$ (20 mL) and filtered. The filtrate was concentrated and purified by flash LC 95/5 EtOAc/MeOH containing (5%) concentrated $NH_4OH$ to give 350 mg of product.

Step 4: N'-[2(S)-cyclopentyl-1(R)-hydroxy-3(R)-methyl]-3(S)-[3(S)-(3(S)-tetrahydrofuranoxy-carbonylamino)-2(S)-hydroxy-4-phenyl-butyl]-3(S)-[4-(2-(4-morpholinyl)ethoxy)-phenyl methyl]-pyrrolidin-2-one

The product from step 3 (0.310g) was dissolved in 5 mL of $CH_2Cl_2$ and added to a saturated HCl/EtOAc solution (20 mL). The resulting solution was stirred for 2 hours after which time the reaction was judged complete by HPLC. The reaction was concentrated to give 350 mg of the HCl salt. This salt was dissolved in 7 mL of $CH_2Cl_2$ and 3(S)-tetrahydrofuranyl succinimidyl carbonate (0.155 g, 0.679 mmol), followed by triethylamine (0.16 mL, 1.14 mmol) were added. The reaction was stirred for 2 hours, concentrated and purified by flash LC 95/5 $CHCl_3$/MeOH containing (5%) concentrated $NH_4OH$ to give 240 mg of product and 60 mg of mixed fractions. The 60 mg was repurified in the same solvent system, combined and dried over $P_2O_5$ yielding 246 mg of the desired product.
m.p. 52 ° C (dec.)

EXAMPLE 14

Preparation of N'-(2(S)-cyclopentyl-1(R)-hydroxy-3(R)-methyl)-3(S)-[3(S)-[N-(carbobenzyloxycarbonylamino-(L) valinyl)]-3(S)-hydroxy-4-phenyl-butyl]-3(S)-[4-(2-(4-morpholinyl)ethoxy)-phenylmethyl]-pyrrolidin-2-one

Following the procedures of the preceeding Example, the HCl salt from step 4 therein (0.5 g, 0.776 mmol), HOBT (0.167 g, 1.24 mmol), EDC (0.238 g, 1.24 mmol), and CBZ-valine(0.243 g, 0.97 mmol) were combined in a 50 mL round-bottom flask and dissolved in DMF (10 mL). This solution was cooled to 0 ° C and triethylamine (0.36 mL, 2.6 mmol) was added via syringe. The reaction was stirred overnight, gradually coming to room temperature. After 14 hours the reaction was concentrated to dryness and the residue dissolved in EtOAc (50 mL), and washed with $NaHCO_3$ (2 x 20 mL). The organic layer was collected and dried ($MgSO_4$). The aqueous layer was re-extracted with of EtOAc (75 mL) and combined with the first. The crude product (800 mg) was purified by flash LC 93/7 EtOAc/iPrOH containing (5%) concentrated $NH_4OH$ to give 340 mg product.

EXAMPLE 15

Preparation of N'-(2(S)-cyclopentyl-1(R)-hydroxy-3(R)-methyl)-3(S)-[3(S)-(N-(2-quinoylcarbonyl-(L)-valinyl)) - 3(S)-hydroxy-4-phenyl-butyl]-[4-(2-(4-morpholinyl)-ethoxy)phenylmethyl]-pyrrolidon-2-one

The product of the previous example, (0.130 g, 0.161 mmol) was dissolved in 5 mL of EtOH. followed by the addition 10% Pd/C (100 mg). The flask was evacuated under house vacuum and backfilled with $H_2$ - (balloon). This process was repeated three times. After 14 hours the catalyst was filtered off and the solvent removed via rotoevaporator. The residue was azeotroped with toluene (2 x 4 mL) and the resulting amine was dissolved in DMF (5 mL). To this solution was added hydroxy benzotriazole (0.034 mg), EDC (0.049g) and quinaldic acid (0.035 g). The solution was cooled to 0 ° C and $Et_3N$ (0.025 mL) was added via syringe. The solution was stirred overnight with gradual warming to room temperature. After 14 hours the DMF was removed and the residue was dissolved in EtOAc (40 mL). This organic layer was washed with $H_2O$ (20 mL), saturated. $NaHCO_3$ (20 mL), and saturated NaCl (20 mL). The organic layer was collected and dried ($MgSO_4$). The aqueous layer was re-extracted with EtOAc (70 mL). The organic layers were combined and dried ($MgSO_4$). The product was isolated as a mixture of diastereomers by flash LC 95/5 EtOAc/MeOH

containing concentrated $NH_4OH$ (5%) (80 mg).

EXAMPLE 16

Preparation of N'-[2(S)-cyclopentyl-1(R)-hydroxy-3(R)-methyl]-3(S)-[3(S)-[(N-(4-oxo-4H-1-benzopyran-2-carbonyl)-L-valinyl)amino]-2(S)-hydroxy-4-phenylbutyl]-3(S)-phenylmethyl-pyrrolidin-2-one, compound F

Step 1: N'-[2(S)-cyclopentyl-1(R)-hydroxy-3(R)-methyl]-3(S)-[3(S)-(1,1-dimethylethoxycarbonylamino)-2(S)-hydroxy-4-phenyl-butyl]-3(S)-phenylmethyl-pyrrolidin-2-one

(3R, 5S, 1'S)-3-ethanal-3-phenylmethyl-5-(1-((1,1'-dimethylethoxycarbonyl)amino)-2-phenyl-ethyl)-dihydrofuran-2-(3H)-one (4.6 g, 10.6 mmol), prepared as in Example 10, Step 2; 2(S)-amino-3(R)-methyl-1-(R)-cyclopentanol HCl (2 g, 13.8 mmol); and activated 3A molecular sieves (powder), were placed in a flame-dried round-bottom flask equipped with a stir bar. A volume of MeOH (60 mL) was added and this mixture was stirred for 0.4 hours. A quantity of $NaCNBH_3$ (0.8651 g, 13.8 mmol) was added to the mixture followed by glacial acetic acid (25 drops) and the reaction was stirred overnight under an argon atmosphere. After 14 hours, no starting material remained, as seen by HPLC. The solution was filtered through a celite pad and the filtrate was treated with 10% citric acid to pH 2.5-3. The MeOH was then removed via rotoevaporator, the residue was diluted with equal volumes of $CHCl_2/CHCl_3$ (150 mL total), and the solution was adjusted to pH 9.5 with 1N NaOH. The layers were separated and the aqueous layer was further extracted 5 x 80 ml 50% $CHCl_3/CH_2Cl_2$. The organic layers were combined, washed with brine, dried over $MgSO_4$, and concentrated to a foam (6 g). This was combined with another batch (5 g) prepared under identical conditions. The residue was dissolved in toluene (300 ml) and HOBT (0.9 g), and the mixture was stirred at 70 °C overnight. HPLC showed no starting material, so the solution was concentrated. The residue was purified by flash chromatography in 70% EtOAc/hexane to give 9.69 g of desired product (85% yield based on 9.2 g of starting aldehyde).

Step 2: N'-[2(S)-cyclopentyl-1(R)-hydroxy-3(R)-methyl]-3(S)-[3(S)-[(1,1-dimethylethoxycarbonyl) amino]-(L)-valinyl)-2(S)-hydroxy-4-phenylbutyl]-3(S)-phenylmethyl-pyrrolidin2-one

The product from above (9.69 g, 18.0 mmol) was dissolved in 80 ml $CH_2Cl_2$ and cooled to 0 °C. Anhydrous ether was saturated with HCl gas and 150 ml of the resulting solution was added to the $CH_2Cl_2$. The mixture was stirred for 2 hours under argon until the HPLC showed no starting material. The solvents were evaporated, and the residue was dried under vacuum. The HCl salt (6.3904 g, 13.5 mmol), L-BOC-valine (3.819 g, 17.6 mmol), EDC (3.374 g, 17.6 mmol), and HOBT (2.378 g, 17.6 mmol) were dissolved in DMF (80 mL), and the solution was cooled to 0 °C. Triethylamine (3.96 mL, 28.4 mmol) was added via syringe, and the thick suspension was diluted with additional DMF (15 mL). With gradual warming to room temperture, the reaction was stirred overnight under argon until no starting material remained. DMF was concentrated under high vacuum. The resulting yellow oil was diluted with 150 mL (50% $CHCl_2/CHCl_3$); washed with 20 mL 10% citric acid, 20 mL $H_2O$, 20 mL saturated $NaHCO_3$, and 20 mL brine; and dried over $MgSO_4$. After filtration, the filtrate was concentrated to a yellow oil which was chromatographed in 50% EtOAc/hexane to give 7.08 g (82.6% yield from HCl salt) of desired product; m.p. 159-160 °C.

Step 3: N'-[2(S)-cyclopentyl-1(R)-hydroxy-3(R)-methyl]-3(S)-[3(S)-[(N-(4-oxo-4H-1-benzo pyran-2-carbonyl)-L-valinyl)amino]-2(S)-hydroxy-4-phenylbutyl]-3(S)-phenylmethylpyrrolidin-2-one

The product from above (7.08 g, 11 mmol) was dissolved in 30 mL $CH_2Cl_2$ and cooled to 0 °C under argon. Anhydrous ether was saturated with HCl gas and 30 mL of the resulting solution was added to the $CH_2Cl_2$. The mixture was stirred for 5 hours, adding 40 mL additional saturated $Et_2O$ solution and 30 mL $CH_2Cl_2$, until no more starting material remained. The solvents were concentrated, and the residue was dried under vacuum. The HCl salt (1.998 g, 3.5 mmol), 4-oxo, 4H-1-benzopyran, 2-carboxylic acid (0.8653 g, 4.55 mmol), EDC (0.872 g, 4.55 mmol), and HOBT (0.6143 g, 4.55 mmol), were dissolved in 24 mL DMF and cooled to 0 °C. Triethylamine (1.02 mL, 7.35 mmol) was added dropwise via syringe. Reaction was allowed to warm gradually to room temperature and stirred overnight under argon. HPLC after 14 hours showed no starting material; DMF was evaporated under vacuum. The brown residue was diluted in $CH_2Cl_2/CHCl_3$, washed with 30 mL 10% citric acid, 30 mL $H_2O$, 30 mL saturated $NaHCO_3$, and 30 mL brine, and dried over $MgSO_4$. After filtration, the filtrate was concentrated to a brown oil. The residue was purified once by MPLC (65% EtOAc/hexane), twice by flash chromatography (70% EtOAc/hexane), and

1.085 g total of desired product was obtained (56.2% yield from HCl salt); m.p.-118-123°C; Partial NMR ($CDCl_3$, 400 MHz) : 4.37 (m, 1H), 0.986 (d, 3H, J=6.78Hz), 0.980 (d, 3H, J=6.96 Hz), 0.876 (d, 3H, J=6.59 Hz). C,H,N analysis calculated for $C_{42}H_{49}N_3O_7$: (707.875):

| | | | |
|---|---|---|---|
| | C (71.27), | H (6.98), | N (5.94). |
| Found | C (71.04), | H (7.03), | N (6.04). |

Assay for Inhibition of Microbial Expressed Viral Protease

Inhibition studies of the reaction of the protease expressed in Escherichia coli with a peptide substrate [Val-Ser-Gln-Asn-(betanapthyl)Ala-Pro-Ile-Val, 0.5 mg/mL at the time the reaction is initiated] were in 50 mM Na acetate, pH 5.5, at 30°C for 1 hour. Various concentrations of inhibitor in 1.0 µl DMSO were added to 25 µl of the peptide solution in water. The reaction is initiated by the addition of 15 µl of 0.33 nM protease (0.11 ng) in a solution of 0.133 M Na acetate pH 5.5 and 0.1% bovine serum albumin. The reaction was quenched with 160 µl of 5% phosphoric acid. Products of the reaction were separated by HPLC (VYDAC wide pore 5 cm C-18 reverse phase, acetonitrile gradient, 0.1% phosphoric acid). The extent of inhibition of the reaction was determined from the peak heights of the products. HPLC of the products, independently synthesized, proved quantitation standards and confirmation of the product composition. The compounds of this invention have $IC_{50}$ values in the range of about 0.1nM - 100µm. The most preferred compounds (A-H) have $IC_{50}$ values of between about 0.2 nM and about 10nM.

While foregoing specification teaches the principles of the present invention, with examples provided for the purpose of illustration, it will be understood that the practice of the invention emcompasses all of the usual variations, adaptations, or modifications, as come within the scope of the following claims and its equivalents.

**Claims**

**1.** A compound of the formula:

A-B-G-J I,

wherein A is:

1) trityl,

2) hydrogen;

3)

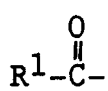

wherein $R^1$ is

a) hydrogen,

b) $C_{1-4}$ alkyl, substituted with one or more halogens adjacent to the carbonyl carbon where halogen is F, Cl, Br, and I,

c) aryl unsubstituted or substituted with one or more of

i) $C_{1-4}$ alkyl,

ii) $C_{1-3}$ alkoxy,

iii) halo,

iv) nitro,

v) acetoxy,

vi) dimethylaminocarbonyl,

vii) phenyl,

viii) $C_{1-3}$ alkoxycarbonyl, or

ix) hydroxy,

d) fluorenyl,

e) a 5-7 membered heterocycle, unsubstituted or substituted with one or more of

i) $C_{1-4}$ alkyl,
ii) $C_{1-3}$ alkoxy,
iii) halo,
iv) nitro,
v) acetoxy,
vi) dimethylaminocarbonyl,
vii) phenyl,
viii) $C_{1-3}$ alkoxycarbonyl, or
ix) hydroxy,

f) indole, quinolyl, naphthyl, benzofuryl, or 4-oxo-benzopyranyl,

g) a 5-7 membered cycloalkyl group unsubstituted or substituted with one or more of

i) $C_{1-4}$ alkyl,
ii) $C_{1-3}$ alkoxy,
iii) halo,
iv) nitro,
v) acetoxy,
vi) dimethylaminocarbonyl,
vii) phenyl,
viii) $C_{1-3}$ alkoxycarbonyl, or
ix) hydroxy,

4) phthaloyl wherein the aromatic ring is unsubstituted or substituted with one or more of

a) $C_{1-4}$ alkyl,
b) halo,
c) hydroxy,
d) nitro,
e) $C_{1-3}$ alkoxy,
f) $C_{1-3}$ alkoxycarbonyl,
g) cyano,
h)

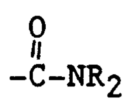

wherein R is H or $C_{1-4}$ alkyl;

5)

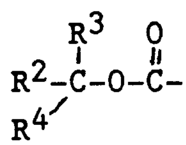

wherein $R^2$,$R^3$, and $R^4$ are independently

a) H,

b) $C_{1-6}$ alkyl unsubstituted or substituted with one or more of

i) halo,
ii) alkyl $SO_2$-,
iii) aryl $SO_2$-,

c) Aryl unsubstituted or substituted with one or more of

i) $C_{1-4}$ alkyl,
ii) $C_{1-3}$ alkoxy,
iii) halo,
iv) nitro,
v) acetoxy,
vi) dimethylaminocarbonyl,
vii) phenyl,

viii) $C_{1-3}$ alkoxycarbonyl

d) fluorenyl,

e) $R^2$, $R^3$, and $R^4$ may be independently joined to form a monocyclic, bicyclic, or tricyclic ring system which is $C_{3-10}$ cycloalkyl and may be substituted with $C_{1-4}$ alkyl,

f) a 5-7 membered heterocycle such as pyridyl, furyl, or benzisoxazolyl;

6)

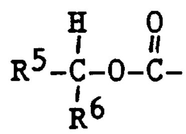

wherein $R^5$ and $R^6$ are

a) $C_{1-4}$ alkyl,

b) aryl,

c) $R^5$ and $R^6$ are joined to form a 5-7 membered heterocycle;

7)

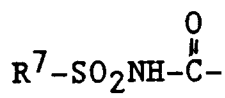

wherein $R^7$ is aryl unsubstituted or substituted with one or more of

a) $C_{1-4}$ alkyl,

b) halo,

c) nitro,

d) $C_{1-3}$ alkoxy;

8)

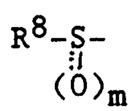

wherein m is 0-2 and $R^8$ is

a) $R^7$ as defined above,

b) trityl;

9)

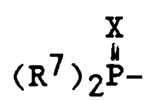

wherein X is O, S or NH, and $R^7$ is defined above;

B is, independently, absent or

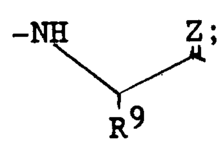

G is

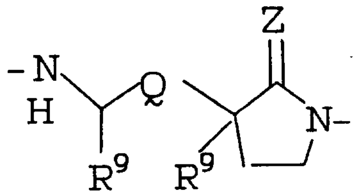

wherein Z is 0, S, or NH and
Q is

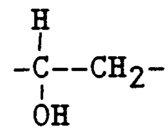

or

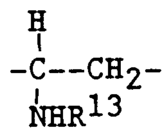 ;

$R^9$ is independently

1) hydrogen;
2)

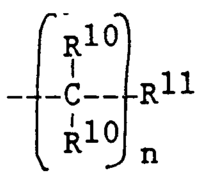 ;

3) $-OR^{11}$,
4) $-N(R^{11})_2$,
5) $C_{1-4}$ alkylene-$R^{11}$ or
6) $-S(R^{11})$;

wherein n is 0-5 and $R^{10}$ is independently

a) hydrogen,
b) hydroxy, or
c) $C_{1-4}$-alkyl;

$R^{11}$ is

a) hydrogen,
b) $C_6$-$C_{10}$ aryl, unsubstituted or substituted with one or more of
   i) halo,
   ii) hydroxy,
   iii) $-NH_2$, $-NO_2$, -NHR, or $-NR_2$,
   wherein R is
   H, or $C_{1-4}$ alkyl,
   iv) $C_{1-4}$ alkyl,
   v) $C_{1-3}$ alkoxy,
   vi) -COOR,
   vii)

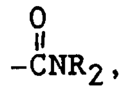

viii)

$-CH_2NR_2$,

O

ix) $-CH_2NHCR$,

x) CN,

xi) $CF_3$,

xii)

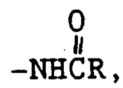

xiii) aryl $C_{1-3}$ alkoxy,

xiv) aryl,

xv) $-NRSO_2R$,

xvi) $-OP(O)(OR_x)_2$ wherein $R_x$ is H or aryl,

xvii)

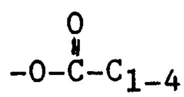

alkyl substituted with one or more of amine or quaternary amine, or

xviii) $-R^{12}$, as defined below;

c) 5 or 6 membered heterocycle including up to 3 heteroatoms selected from N, O, and S, any of which heterocycle may be unsubstituted or substituted with one or more of

i) halo,

ii) hydroxy,

iii) $-NH_2$, $-NHR$, $-NR_2$,

iv) $C_{1-4}$ alkyl,

v) $C_{1-3}$ alkoxy,

vi) -COOR,

vii)

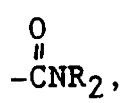

viii) $-CH_2NR_2$,

ix)

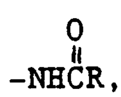

x) -CN,

xi) $CF_3$,

xii) $-NHSO_2R$,

xiii) $-OP(O)(OR_x)_2$ wherein $R_x$ is H or aryl,

xiv)

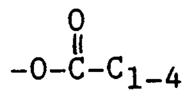

alkyl substituted with one or more of amine or quaternary amine, or
xv) -$R^{12}$;

d) $C_{1-6}$ alkyl or $C_{1-6}$ alkenyl, unsubstituted or substituted with one or more of
i) hydroxy,
ii) $C_{1-4}$ alkyl,
iii) $-NH_2$, -NHR, $-NR_2$,
iv)

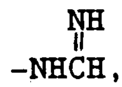

v)

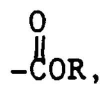

vi) -SR, or arylthio,
xi) $-SO_2NHR$,
vii) $C_{1-4}$ alkyl sulfonyl amino or aryl sulfonyl amino,
viii) -CONHR,
ix)

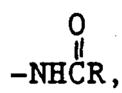

x) -OR,
xi) aryl $C_{1-3}$ alkoxy,
xii) aryl, or
xiii) aryl substituted with $R^{12}$;

e) $C_{3-7}$ cycloalkyl unsubstituted or substituted with one or more of
i) hydroxy,
ii) $C_{1-4}$alkyl,
iii) $-NH_2$, -NHR, $-NHR_2$,
iv)

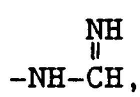

v)

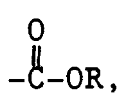

vi) -SR,
vii) $-SO_2NH_2$,

viii) alkyl sulfonylamino or aryl sulfonylamino,
ix) -CONHR,
x)

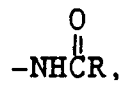

or
xi) $-R^{12}$;

f) a 5- to 7-membered carbocyclic or 7- to 10-membered bicyclic carbocyclic ring which is either saturated or unsaturated, the carbocyclic ring being unsubstituted or substituted with one or more of
i) halo
ii) -OR, wherein R is H or $C_{1-4}$ alkyl,
iii)

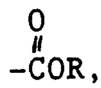

iv)

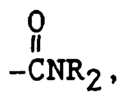

v) $-CH_2NR_2$,
vi) $-SO_2NR_2$ or $-S(O)_y$ R wherein y is 0,1 or 2,
vii) $-NR_2$,
viii)

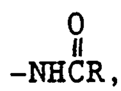

ix) $C_{1-4}$ alkyl,
x) phenyl,
xi) $-CF_3$,
xii)

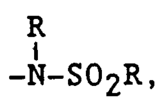

or
xiii) $-R^{12}$;

$R^{12}$ is
a) $-X-(CH_2)_m--XR^{13}$ where X is independently -O-,-S-, or NR; m is 2-5, and $R^{13}$ is independently hydrogen or
i) $C_{1-6}$ alkyl,
ii) $C_{1-6}$ alkyl substituted with one or more of
(a) $C_{1-3}$ alkoxy,
(b) -OH,
(c) $-NR_2$ where R is hydrogen Or $C_{1-4}$ alkyl;
iii) aromatic heterocycle unsubstituted or substituted with one or more of
(a) $C_{1-4}$ alkyl, or
(b) $-NR_2$;

b) $-X-(CH_2)_m-NR^{13}R^{13}$ wherein $R^{13}$ is the same or different and joined together to form a 5-7 member heterocycle containing up to two additional heteroatoms selected from

(a) -NR,

(b) -O-,

(c) -S-,

(d)

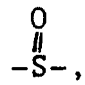

(e) $-SO_2-$;

c) $-(CH_2)_q--NR^{13}R^{13}$ wherein q is 1-5, and $R^{13}$ is defined above;

J is

1) $R^{14}$ wherein:

$R^{14}$ is

a) H;

b) $C_{1-6}$ alkyl, unsubstituted or substituted with one or more of

i) $-NR_2$,

ii) -OR,

iii) $-NHSO_2C_{1-4}$ alkyl,

iv) $-NHSO_2$ aryl, or $-NHSO_2$(dialkyl-aminoaryl),

v) $-CH_2OR$,

vi) $-C_{1-4}$ alkyl,

vii)

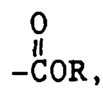

viii)

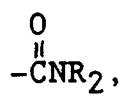

ix)

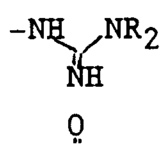 ; 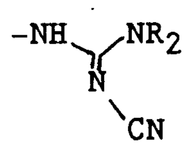

x)

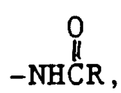

xi)

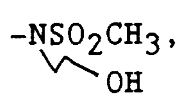

xii)

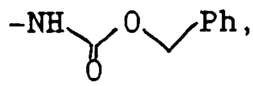

xiii) $-NR_3^{\oplus} A^{\ominus}$ wherein $A^{\ominus}$ is a counterion,
xiv) $-NR^{15}R^{16}$ wherein $R^{15}$ and $R^{16}$ are the same or different and are $C_{1-5}$ alkyl joined together directly to form a 5-7 membered heterocycle,
xv) aryl,
xvi) -CHO,
xvii) $-OP(O)(OR_x)_2$ wherein $R_x$ is H or aryl, or xvii)

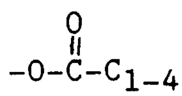

alkyl substituted with one or more of amine or quaternary amine;
c) $-(CH_2CH_2O)_nCH_3$ or $-(CH_2CH_2O)_n H$;
2)

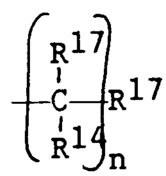 ;

wherein:
$R^{14}$ and n are defined above, and
$R^{17}$ is a) hydrogen;
b) aryl unsubstituted or substituted with one or more of
i) halo,
ii) -OR, wherein R is H or $C_{1-4}$ alkyl,
iii)

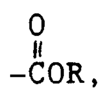

iv)

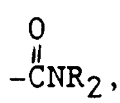

v) $-CH_2NR_2$,
vi) $-SO_2NR_2$,
vii) $-NR^2_2$ ,
viii)

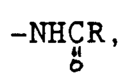

xi) $C_{1-4}$ alkyl,
x) phenyl
xi) $-CF_3$,
xii)

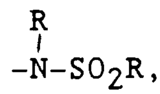

xiii) $-C_{1-4}$ alkyl $-NR_2$,
xiv) $-OP(O)(OR_x)_2$ wherein $R_x$ is H or aryl, or
xv)

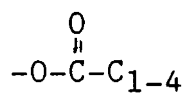

alkyl substituted with one or more of amine or quaternary amine;

c) Heterocycle unsubstituted or substituted with one or more of
i) halo,
ii) -OR, wherein R is H, $C_{1-4}$alkyl, or $C_{1-4}$alkenyl,
iii)

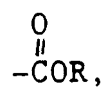

iv)

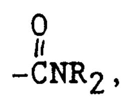

v) $-CH_2NR_2$,
vi) $-SO_2NR_2$,
vii) $-NR_2$,
viii)

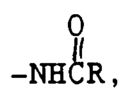

xi) $C_{1-4}$ alkyl,
x) phenyl
xi) $-CF_3$,
xii)

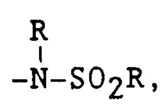

xiii) phenyl $C_{1-4}$ alkyl,
xiv) $-OP(O)(OR_x)2$ wherein $R_x$ is H or aryl,
xv)

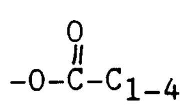

alkyl substituted with one or more of amine or quaternary amine;

d) A 5 to 7 membered carbocyclic or 7-10 membered bicyclic carbocyclic ring which is either saturated or unsaturated, the carbocyclic ring being unsubstituted or substituted with one or more of

i) halo,
ii) -OR, wherein R is H or $C_{1-4}$ alkyl,
iii)

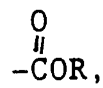

iv)

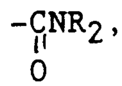

v) $-CH_2NR_2$,
vi) $-SO_2NR_2$,
vii) $-NR_2$,
viii)

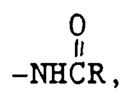

xi) $C_{1-4}$ alkyl,
x) phenyl
xi) $-CF_3$,
xii)

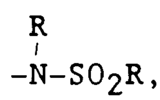

xiii) $-OP(O)(OR_x)_2$ wherein $R_x$ is H or aryl, or
xiv)

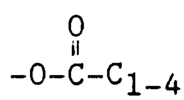

alkyl substituted with one or more of amine, quaternary amine, or $-OP(O)(OR_x)_2$; or
xv)

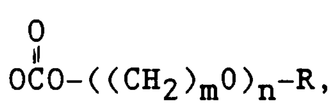

or pharmaceutically acceptable salts thereof.

**2.** A compound of Claim 1 wherein B is independently present once and Z is O.

**3.** A compound of Claim 2 wherein Q is

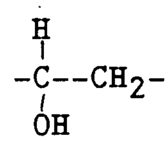 ;

4. A compound of Claim 1 wherein B is absent.

5. A compound of Claim 4 wherein Q is

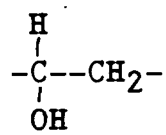 ;

6. A compound of Claim 1 wherein G is

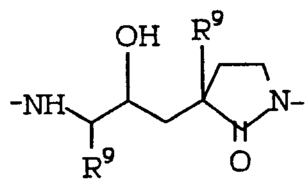

or

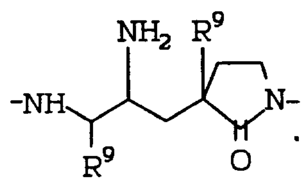

7. A compound of Claim 1 wherein G is.

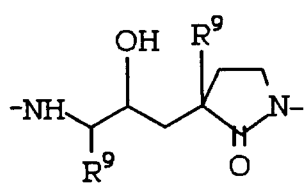

or

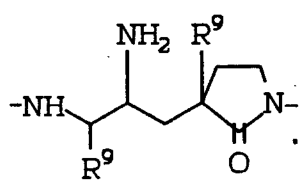

B is absent or present once, and
J is

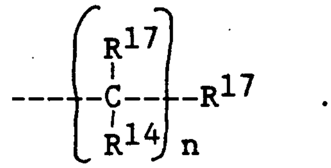

8. A compound of Claim 1 wherein
A is

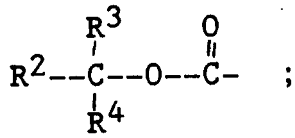

G is

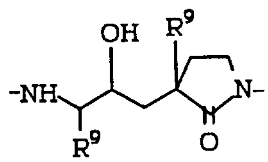

or

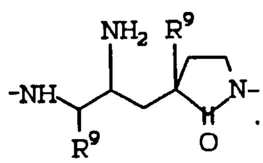

B is absent or present once, and
J is

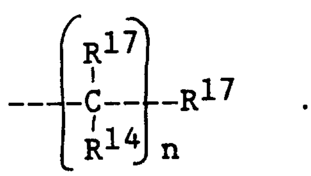

9. A compound of Claim 1 wherein
A is

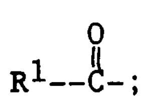

G is

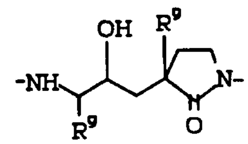

B is absent or present once, and
J is

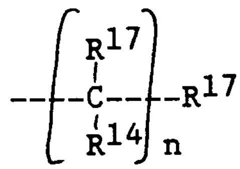

.

**10.** A compound, which is
N'-[4(RS)-(3,4-dihydro-1H-2-benzothiopyranylsulfoxide)]-3(S)-[3(S)-((1,1-dimethylethoxycarbonyl)-amino)-2(S)-hydroxy-4-phenylbutyl]-3(S)-(phenylmethyl)pyrrolidin-2-one,
N'-[4(R)-(3,4-dihydro-1H-2-benzothiopyranylsulfone)]-3(S)-[3(S)-((1,1-dimethylethoxycarbonyl)amino)-2-(S)-hydroxy-4-phenylbutyl]-3(S)-(phenylmethyl)-pyrrolidin-2-one,
N'-[4(S)-(3,4-dihydro-1H-2-benzothiopyranylsulfone)]-3(S)-[3(S)-(3(S)-tetrahydrofuranoxycarbonylamino)-2-(S)-hydroxy-4-phenylbutyl]-3(S)-(phenylmethyl)-pyrrolidin-2-one,
N'-[4(RS)-(3,4-dihydro-1H-2-benzothiopyranyl)]-3(S)-[3(S)-((1,1-dimethylethoxycarbonyl)amino)-2(S)-hydroxy-4-cyclohexylbutyl]-3(S)-(4-hydroxyphenyl-methyl)-pyrrolidin-2-one,
N'-[4(RS)-(3,4-dihydro-1H-2-benzothiopyranyl)]-3(S)-[3(S)-(1,1-dimethylethoxycarbonylamino)-2(S)-hydroxy-4-(cyclohexyl)butyl]-3(S)-(phenylmethyl)-pyrrolidin-2-one,
N'-[4(S)-(3,4-dihydro-1H-2-benzothiopyranylsulfide)]-3(S)-[3(S)-(3(S)-tetrahydrofuranoxycarbonylamino)-2(S)-hydroxy-4-(cyclohexyl)butyl]-3(S)-(phenylmethyl)-pyrrolidin-2-one,
N'-[4(R)-(3,4-dihydro-1H-2-benzothiopyranylsulfide)]-[3(S)-(3(S)-tetrahydrofuranoxycarbonylamino)-2(S)-hydroxy-4-(phenyl)-butyl]-3(S)-(phenylmethyl)-pyrrolidinone,
N'-[4(RS)-(3,4-dihydro-1H-2-benzothiopyranyl)]-3(S)-[3(S)-(1,1-dimethylethoxycarbonylamino)-2(S)-hydroxy-4-(cyclohexyl)-butyl]-3(S)-((4-(2-(4-morpholino)-ethoxy)phenyl)methyl)-pyrrolidin-2-one,
N'-[4(R)-(cis(3-hydroxy-1-indanyl))]-3(S)-[3(S)-(3(S)-tetrahydrofuranoxycarbonylamino)-2(S)-hydroxy-4-phenylbutyl]-3(S)-(phenylmethyl)-pyrrolidin-2-one,
N'-[4(S),2(R)-(3,4-dihydro-1H-2-benzooxothiopyranyl)]-3(S)-[3(S)-(3(S)-tetrahydrofuranoxycarbonylamino)-2-(S)-hydroxy-4-(cyclohexyl)butyl]-3(S)-(phenylmethyl)-pyrrolidin-2-one,
N'-[1-hydroxy-3-methyl-2-cyclopentyl]-3(S)-[3(S)-(1,1-dimethylethoxycarbonylamino)-2(S)-hydroxy-4-phenylbutyl]-3(S)-(phenylmethyl)-pyrrolidin-2-one,
N'-[(4S)-(3,4-dihydro-1H-2-benzothiopyranyl)]-3(S)-[3(S)-(3(S)-tetrahydrofuranyloxylcarbonylamino)-2(S)-hydroxy-4-(cyclohexyl)butyl]-3(S)-((4-(2-(4-morpholino)ethoxy)phenyl)methyl)-pyrrolidin-2-one,
N'-[(4S),(2S)-(3,4-dihydro-1H-2-benzooxothiopyranyl)]-3(S)-[3(S)-(3(S)-tetrahydrofuranyloxycarbonylamino)-2(S)-hydroxy-4-(cyclohexyl)-butyl]-3(S)-(4-hydroxyphenylmethyl)-pyrrolidin-2-one,
N'-[(4S),(2RS)-(3,4-dihydro-1H-2-benzooxothiopyranyl)] -3(S)-[3(S)-3(S)-tetrahydrofuranyloxycarbonylamino)-2(S)-hydroxy-4-(cyclohexyl)-butyl]-3(S)-((4-(2-(4-morpholino)ethoxy)phenyl)methyl)-pyrrolidin-2-one,
N'-[1-hydroxy-3-methyl-2-cyclopentyl]-3(S)-[3(S)-(3(S)-tetrahydrofuranyloxycarbonylamino)-2(S)-hydroxy-4-phenylbutyl]-3(S)-(phenylmethyl)-pyrrolidin-2-one,
N'-[2(S)-isopropylethanol]-3(S)-[3(S)-(3(S)-tetra-hydrofuranoxycarbonylamino)-2(S)-hydroxy-4-(phenyl)-butyl]-3(S)-(phenylmethyl)-pyrrolidin-2-one,
N'-[(5S, 1'S)-(2-(methyl)ethyl-dihydrofuran-2-(3H)-one)yl]-3(S)-[3(S)-[N-(3(S)-tetrahydrofuranoxycarbonyl)-amino]-2(S)-hydroxy-4-(phenyl)butyl]-3(S)-phenylmethyl-pyrrolidin-2-one,
N'-[2(S)-isopropylethanol]-3(S)-[3(S)-[N-(4(R)-hydroxy-3(S)-tetrahydrofuranoxycarbonyl)amino]-2(S)-hydroxy-4-phenylbutyl]-3(S)-phenylmethyl-pyrrolidin-2-one,
N'-[1(R)-hydroxy-3(R)-methyl-2-cyclopentyl]-3(S)-[3(S)-[N-(3(S)-tetrahydrofuranoxycarbonyl)amino]-2(S)-hydroxy-4-phenylbutyl]-3(S)-phenylmethyl-pyrrolidin-2-one,
N'-[1(R)-hydroxy-3(R)-methyl-2-cyclopentyl]-3(S)-[(3(S)-[N-(3(S)-tetrahydrofuranoxycarbonyl)amino]-2-(S)-hydroxy-4-(cyclohexyl)butyl]-3(S)-((4-(2-(dimethylamino)ethoxy)phenyl)methyl)-pyrrolidin-2-one,
N'-[1(R)-hydroxy-3(R)-methyl-2-cyclopentyl]-3(S)-[(3(S)-[N-(3(S)-tetrahydrofuranoxycarbonyl)amino]-2-(S) -hydroxy-4-(cyclohexyl)butyl]-3(S)-((4-(2-(4-morpholino)ethoxy)phenyl)methyl)-pyrrolidin-2-one,
N'-[1(R)-hydroxy-3(R)-methyl-2-cyclopentyl]-3(S)-[3-(S)-[N-(3(S)-tetrahydrofuranoxycarbonyl)amino]-2-(S)-hydroxy-4-(cyclohexyl)butyl]-3(S)-((4-(2-(3,6,9,12-tetraoxatridecyloxy)phenyl)methyl)-pyrrolidin-2-one,
N'-[1(R)-hydroxy-3(R)-methyl-2-cyclopentyl]-3(S)-[3-(S)-[(N-(benzyloxycarbonyl)-L-Valinyl)amino]-2(S)-hydroxy-4-phenylbutyl]-3(S)-phenylmethyl-pyrrolidin2-one,
N'-[1(R)-hydroxy-3(R)-methyl-2-cyclopentyl]-3(S)-[3-(S)-[(N-(2-quinolylcarbonyl)-L-Valinyl)amino]-2(S)-hydroxy-4-phenylbutyl]-3(S)-phenylmethyl-pyrrolidin-2-one,
N'-[1(R)-hydroxy-3(R)-methyl-2-cyclopentyl]-3(S)-[3-(S)-[(N-(benzyloxycarbonyl)-L-Valinyl)amino]-2(S)-hydroxy-4-(cyclohexyl)butyl]-3(S)-((4-(2-(4-morpholino)ethoxy)phenyl)methyl)-pyrrolidin-2-one,
N'-[1(R)-hydroxy-3(R)-methyl-2-cyclopentyl]-3(S)-[3-(S)-[(N-(2-quinolylcarbonyl)-L-valinyl)amino]-2(S)-hydroxy-4-(cyclohexyl)butyl]-3(S)-((4-(2(4-morpholino)ethoxy)phenyl)methyl)-pyrrolidin-2-one,
N'-[1(R)-hydroxy-3(R)-methyl-2-cyclopenty1]-3(S)-[3-(S)-[(N-(4-pyridylcarbonyl)-L-valinyl)amino]-2(S)-hydroxy-4-phenylbutyl]-3(S)-phenylmethyl-pyrrolidin-2-one,
N'-[1(R)-hydroxy-3(R)-methyl-2-cyclopentyl]-3(S)-[3-(S)-[(N-(3-pyridylcarbonyl)-L-valinyl)amino]-2(S)-hydroxy-4-phenylbutyl]-3(S)-phenylmethyl-pyrrolidin-2-one,
N'-[1(R)-hydroxy-3(R)-methyl-2-cyclopentyl]-3(S)-[3-(S)-[(N-(4-oxo-4H-1-benzopyran-2-carbonyl)-L-valinyl)-amino]-2(S)-hydroxy-4-phenylbutyl]-3(S)-phenylmethyl-pyrrolidin-2-one,
N'-[1(R)-hydroxy-3(R)-methyl-2-cyclopentyl]-3(S)-[3-(S)-[(N-(4-morpholinocarbonyl)-L-valinyl)amino]-2-(S)-hydroxy-4-phenylbutyl]-3(S)-phenylmethyl-pyrrolidin-2-one,
N'-[1(R)-hydroxy-3(R)-methyl-2-cyclopentyl]-3(S)-[3-(S)-[(N-(3-pyridylcarbonyl)-L-valinyl)amino]-2(S)-hydroxy-4-(cyclohexyl)butyl]-3(S)-((4-(2-(4-morpholino)ethoxy)phenyl)methyl)-pyrrolidin-2-one,
N'-[1(R)-hydroxy-3(R)-methyl-2-cyclopentyl]-3(S)-[3-(S)-[(N-(4-oxo-4H-1-benzopyran-2-carbonyl)-L-valinyl)-amino]-2(S)-hydroxy-4-(cyclohexyl)butyl]-3(S)-((4-(2-(4-morpholino)ethoxy)phenyl)methyl-pyrrolidin-2-one,
N'-[(5S, 1'S)-(2-(methyl)ethyl-dihydrofuran-2-(3H)-one)yl]-3(S)-[3(S)-[(N-(4-oxo-4H-1-benzopyran-2-carbonyl)-L-valinyl)amino]-2(S)-hydroxy-4-(cyclohexyl)-butyl]-3(S)-((4-(2-(4-morpholino)ethoxy)phenyl)-methylpyrrolidin-2-one, or
N'-[(5S, 1'S)-(2-(methyl)ethyl-dihydrofuran-2-(3H)-one)yl]-3(S)-[3(S)-[(N-(3-pyridylcarbonyl)-L-valinyl)-amino]-2(S)-hydroxy-4-(cyclohexyl)butyl]-3(S)-phenylmethylpyrrolidin-2-one,
or pharmaceutically acceptable salt or ester thereof.

**11.** A compound, which is
N'-[4(S),2(R)-(3,4-dihydro-1H-2-benzooxothiopyranyl)]-3(S)-[3(S)-(3(S)-tetrahydrofuranoxycarbonyl-amino)-2-(S)--Hydroxy-4-(cyclohexyl)butyl]-3(S)-(phenylmethyl)-pyrrolidin-2-one,
N'-[4(S)-(3,4-dihydro-1H-2-benzothiopyranylsulfone)]-3(S)-[3(S)-(3(S)-tetrahydrofuranoxycarbonyl-amino)-2-(S)-hydroxy-4-(phenylbutyl)]-3(S)-(phenylmethyl)-pyrrolidin-2-one,
N'-[(5S,1'S)-(2-(methyl)ethyl-dihydrofuran-2-(3H)-one)yl]-3(S)-[3(S)-[(N-(3-pyridylcarbonyl)-L-valinyl) amino]-2(S)-hydroxy-4-(cyclohexyl)butyl]-3(S)-phenylmethyl-pyrrolidin-2-one,
N'-[1(R)-hydroxy-3(R)-methyl-2-cyclopentyl]-3(S)-[3-(S)-[(N-(3-pyridylcarbonyl)-L-valinyl)amino]-2(S)-hydroxy-4-(cyclohexyl)butyl]-3(S)-((4-(2-(4-morpholino) ethoxy)phenyl)methyl)-pyrrolidin-2-one,
N'-[1(R)-hydroxy-3(R)-methyl-2-cyclopentyl]-3(S)-[3-(S)-[(N-(4-morpholinocarbonyl)-L-valinyl)amino]-2-(S)-hydroxy-4-phenylbutyl]-3(S)-phenylmethyl-pyrrolidin-2-one,
N'-[1(R)-hydroxy-3(R)-methyl-2-cyclopentyl]-3(S)-[3(S) -[(N-(4-oxo-4H-1-benzopyran-2-carbonyl)-L-valinyl) amino]-2(S)-hydroxy-4-phenylbutyl]-3(S)-phenylmethyl -pyrrolidin-2-one,
N'-[1(R)-hydroxy-3(R)-methyl-2-cyclopentyl]-3(S)-[3(S) -[N-(3(S)-tetrahydrofuranoxycarbonyl)amino]-2-(S) -hydroxy-4-(cyclohexyl)butyl]-3(S)-((4-(2-(4-morpholino) ethoxy)phenyl)methyl)-pyrrolidin-2-one,
N'-[(5S,1'S)-(2-(methyl)ethyl-dihydrofuran-2-(3H)-one) yl]-3(S)-[3(S)-[N-(3(S)-tetrahydrofuranoxycarbonyl) amino]-2(S)-hydroxy-4-(phenyl)butyl]-3(S)-phenylmethyl -pyrrolidin-2-one,
or pharmaceutically acceptable salt thereof.

**12.** A pharmaceutical composition comprising a compound as in any one of claims 1-11, and a pharmaceutically acceptable carrier.

**13.** The use of a compound as claimed in any of claims 1-11 for the manufacture of a medicament for inhibiting HIV protease.

**14.** The use of a compound as claimed in any of claims 1-11 for the manufacture of a medicament for preventing or treating infection by HIV, AIDS, or ARC.

European Patent Office

# EUROPEAN SEARCH REPORT

Application Number

EP 92 20 3810

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 356 223 (MERCK) 28 February 1990 * the whole document * | 1-14 | C07D207/26 C07D401/04 C07D401/12 C07D403/12 C07D405/04 C07D405/12 C07D405/14 C07D409/04 C07D409/12 C07D409/14 |
| A | EP-A-0 337 714 (MERCK) 18 October 1989 * the whole document * | 1-14 | |
| A | EP-A-0 434 365 (MERCK) 26 June 1991 * the whole document * | 1-14 | |

TECHNICAL FIELDS SEARCHED (Int. Cl.5)

C07D

The present search report has been drawn up for all claims

| Place of search | Date of completion of the search | Examiner |
|---|---|---|
| THE HAGUE | 07 APRIL 1993 | Bernd Kissler |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)